# EUROPEAN PATENT SPECIFICATION

(11) **EP 1 585 832 B1**
(45) Date of publication and mention of the grant of the patent: **13.02.2008**
(21) Application number: 03790313.5
(22) Date of filing: 05.12.2003
(51) Int. Cl.: C12Q 1/68, C07H 21/00, C07H 21/02

(54) **COMPOSITIONS AND METHODS FOR POLYNUCLEOTIDE DETECTION**
ZUSAMMENSETZUNGEN UND VERFAHREN FÜR DEN NACHWEIS VON POLYNUKLEOTIDEN
COMPOSITIONS ET PROCEDES DE DETECTION DE POLYNUCLEOTIDES

(30) Priority: 20.12.2002 US 435484 P
(43) Date of publication of application: 19.10.2005
(73) Proprietor: STRATAGENE, La Jolla, CA 92037 (US)
(72) Inventor: SORGE, Joseph, A., California 92014 (US); SUN, Gulan, Austin, Texas 78759 (US); MUELLER, Reinhold, Dietrich, San Diego, CA 92131 (US)
(74) Representative: De Clercq, Ann G. Y.
(86) International application number: PCT/US2003/038501
(87) International publication number: WO 2004/061132

(56) References cited:
- EP-A2- 0 229 943
- US-A- 5 723 591
- US-A- 6 037 130
- US-B1- 6 350 580
- SEI-IIDA Y ET AL: "Real-time monitoring of in vitro transcriptional RNA synthesis using fluorescence resonance energy transfer." NUCLEIC ACIDS RESEARCH. 15 JUN 2000, vol. 28, no. 12, 15 June 2000 (2000-06-15), page E59, XP002372128 ISSN: 1362-4962
- LI QINGGE ET AL: "A new class of homogeneous nucleic acid probes based on specific displacement hybridization." NUCLEIC ACIDS RESEARCH. 15 JAN 2002, vol. 30, no. 2, 15 January 2002 (2002-01-15), page E5, XP002372129 ISSN: 1362-4962
- BAGWELL ET AL.: 'A new homogeneous assay system for specific nucleic acid sequences: poly-dA and poly-A detection' NUCLEIC ACIDS RESEARCH vol. 22, no. 12, 1994, pages 2424 - 2425, XP001146995

### SEQUENCE LISTING

<110> Stratagene
<120> COMPOSITIONS AND METHODS FOR POLYNUCLEOTIDE DETECTION
<130> 25436/2352
<150> US 60/435,484
   <151> 2002-12-20
<160> 10
<170> PatentIn version 3.2
<210> 1
   <211> 53
   <212> DNA
   <213> Artificial Sequence
<220>
   <223> Synthetic primer
<220>
   <221> misc_feature
   <222> (1)..(53)
   <223> Synthetic primer
<400> 1
   tgggcaagcc attgagtgga tctaatgaca ggtagaagac gtgctctagt tac 53
<210> 2
   <211> 29
   <212> DNA
   <213> Artificial Sequence
<220>
   <223> Synthetic primer
<220>
   <221> misc_feature
   <222> (1) .. (29)
   <223> Synthetic primer
<400> 2
   gtaactagag cacgtcttct acctgtcat 29
<210> 3
   <211> 50
   <212> DNA
   <213> Artificial Sequence
<220>
   <223> Synthetic primer
<220>
   <221> misc_feature
   <222> (1)..(50)
   <223> Synthetic primer
<400> 3
   tctgacgtta tactcggtta cggaagtttt gacccggagg ctttcacaga 50
<210> 4
   <211> 27
   <212> DNA
   <213> Artificial Sequence
<220>
   <223> Synthetic primer
<220>
   <221> misc_feature
   <222> (1) .. (27)
   <223> Synthetic primer
<400> 4
   acttccgtaa ccgagtataa cgtcaga 27
<210> 5
   <211> 19
   <212> DNA
   <213> Artificial Sequence
<220>
   <223> Synthetic primer
<220>
   <221> misc_feature
   <222> (1)..(19)
   <223> Synthetic primer
<400> 5
   cccagactta cagcaccag 19
<210> 6
   <211> 20
   <212> DNA
   <213> Artificial Sequence
<220>
   <223> Synthetic primer
<220>
   <221> misc_feature
   <222> (1)..(20)
   <223> Synthetic primer
<400> 6
   gagtccagga gcattttagc 20
<210> 7
   <211> 53
   <212> DNA
   <213> Artificial Sequence
<220>
   <223> Synthetic primer
<220>
   <221> misc_feature
   <222> (1) .. (53)
   <223> Synthetic primer
<400> 7
   tgggcaagcc attgagtgga tctaatgaca ggtagaagac gtgctctagt tac 53
<210> 8
   <211> 29
   <212> DNA
   <213> Artificial Sequence
<220>
   <223> Synthetic primer
<220>
   <221> misc_feature
   <222> (1) .. (29)
   <223> Synthetic primer
<400> 8
   gtaactagag cacgtcttct acctgtcat 29
<210> 9
   <211> 50
   <212> DNA
   <213> Artificial Sequence
<220>
   <223> Synthetic primer
<220>
   <221> misc_feature
   <222> (1)..(50)
   <223> Synthetic primer
<400> 9
   tctgacgtta tactcggtta cggaagtttt gacccggagg ctttcacaga 50
<210> 10
   <211> 27
   <212> DNA
   <213> Artificial Sequence
<220>
   <223> Synthetic primer
<220>
   <221> misc_feature
   <222> (1)..(27)
   <223> Synthetic primer
<400> 10
   acttccgtaa ccgagtataa cgtcaga 27

### SEQUENCE LISTING

<110> Stratagene California
<120> COMPOSITIONS AND METHODS FOR POLYNUCLEOTIDE DETECTION
<130> STRAC-003-EP
<150> US 60/435,484
   <151> 2002-12-20
<160> 10
<170> PatentIn version 3.2
<210> 1
   <211> 53
   <212> DNA
   <213> Artificial Sequence
<220>
   <223> Synthetic primer
<220>
   <221> misc_feature
   <222> (1)..(53)
   <223> Synthetic primer
<400> 1
   tgggcaagcc attgagtgga tctaatgaca ggtagaagac gtgctctagt tac 53
<210> 2
   <211> 29
   <212> DNA
   <213> Artificial sequence
<220>
   <223> Synthetic primer
<220>
   <221> misc_feature
   <222> (1)..(29)
   <223> Synthetic primer
<400> 2
   gtaactagag cacgtcttct acctgtcat 29
<210> 3
   <211> 50
   <212> DNA
   <213> Artificial Sequence
<220>
   <223> Synthetic primer
<220>
   <221> misc_feature
   <222> (1)..(50)
   <223> Synthetic primer
<400> 3
   tctgacgtta tactcggtta cggaagtttt gacccggagg ctttcacaga 50
<210> 4
   <211> 27
   <212> DNA
   <213> Artificial Sequence
<220>
   <223> Synthetic primer
<220>
   <221> misc_feature
   <222> (1)..(27)
   <223> Synthetic primer
<400> 4
   acttccgtaa ccgagtataa cgtcaga 27
<210> 5
   <211> 19
   <212> DNA
   <213> Artificial Sequence
<220>
   <223> Synthetic primer
<220>
   <221> misc_feature
   <222> (1)..(19)
   <223> Synthetic primer
<400> 5
   cccagactta cagcaccag 19
<210> 6
   <211> 20
   <212> DNA
   <213> Artificial sequence
<220>
   <223> Synthetic primer
<220>
   <221> misc_feature
   <222> (1)..(20)
   <223> Synthetic primer
<400> 6
   gagtccagga gcattttagc 20
<210> 7
   <211> 53
   <212> DNA
   <213> Artificial sequence
<220>
   <223> Synthetic primer
<220>
   <221> misc_feature
   <222> (1)..(53)
   <223> Synthetic primer
<400> 7
   tgggcaagcc attgagtgga tctaatgaca ggtagaagac gtgctctagt tac 53
<210> 8
   <211> 29
   <212> DNA
   <213> Artificial Sequence
<220>
   <223> Synthetic primer
<220>
   <221> misc_feature
   <222> (1) (29)
   <223> Synthetic primer
<400> 8
   gtaactagag cacgtcttct acctgtcat 29
<210> 9
   <211> 50
   <212> DNA
   <213> Artificial Sequence
<220>
   <223> Synthetic primer
<220>
   <221> misc_feature
   <222> (1)..(50)
   <223> Synthetic primer
<400> 9
   tctgacgtta tactcggtta cggaagtttt gacccggagg ctttcacaga 50
<210> 10
   <211> 27
   <212> DNA
   <213> Artificial sequence
<220>
   <223> synthetic primer
<220>
   <221> misc_feature
   <222> (1)..(27)
   <223> synthetic primer
<400> 10
   acttccgtaa ccgagtataa cgtcaga 27

## Description

### FIELD OF THE INVENTION

This invention relates to a probe system for polynucleotide determination using non-target-hybridizing probes and fluorescence resonance energy transfer.

### BACKGROUND

Techniques for polynucleotide detection have found widespread use in basic research, diagnostics, and forensics. Polynucleotide detection can be accomplished by a number of methods. Most methods rely on the use of the polymerase chain reaction (PCR) to amplify the amount of target DNA.

TaqMan is a homogenous assay for detecting polynucleotides (U.S. Patents 5,723,591). In this assay, two PCR primers flank a central probe oligonucleotide. The probe oligonucleotide contains two fluorescent moieties. During the polymerization step of the PCR process, the polymerase cleaves the probe oligonucleotide. The cleavage causes the two fluorescent moieties to become physically separated, which causes a change in the wavelength of the fluorescent emission. As more PCR product is created, the intensity of the novel wavelength increases.

Molecular beacons are an alternative to TaqMan (U.S. Patent Nos. 6,277,607; 6,150,097; 6,037,130) for the detection of polynucleotides. Molecular beacons are oligonucleotide hairpins which undergo a conformational change upon binding to a perfectly matched template. The conformational change of the oligonucleotide increases the physical distance between a fluorophore moiety and a quencher moiety present on the oligonucleotide. This increase in physical distance causes the effect of the quencher to be diminished, thus increasing the signal derived from the fluorophore.

U.S. Patent NO. 6,174,670B1 discloses methods of monitoring hybridization during a polymerase chain reaction which are achieved with rapid thermal cycling and use of double stranded DNA dyes or specific hybridization probes in the presence of a fluorescence resonance energy transfer pair - fluorescein and Cy5.3 or Cy5.5. The method amplifies the target sequence by polymerase chain reaction in the presence of two nucleic acid probes that hybridize to adjacent regions of the target sequence, one of the probes being labeled with an acceptor fluorophore and the other probe labeled with a donor fluorophore of a fluorescence energy transfer pair such that upon hybridization of the two probes with the target sequence, the donor fluorophore interacts with the acceptor fluorophore to generate a detectable signal. The sample is then excited with light at a wavelength absorbed by the donor fluorophore and the fluorescent emission from the fluorescence energy transfer pair is detected for the determination of that target amount

There are also several other fluorescent and enzymatic PCR technologies, such as Scorpions^{™}, Sunrise^{™} primers, and DNAzymes, for polynucleotide detection, where each polynucleotide to be detected requires a different oligonucleotide probe and two different fluorescent moieties. These probes are usually custom-synthesized and are thus expensive.

### SUMMARY OF THE INVENTION

In one aspect, it is provided a composition comprising (1) a polynucleotide target; (2) a first target-hybridizing probe which comprises a target binding sequence (P1-DNA) which hybridizes to a strand of the target polynucleotide and a probe binding sequence (P1-P); and (3) a second target-hybridizing probe which comprises a target binding sequence (P2-DNA) which hybridizes, in close proximity, to the same strand of the target polynucleotide and a probe binding sequence (P2-P)

In one embodiment, the subject composition of the invention further comprises a non-target-hybridizing universal probe 3 labeled with label A and a non-target-hybridizing universal probe 4 labeled with label B, where the universal probe 3 hybridize to the P1-P sequence and the universal probe 4 hybridizes to the P2-P sequence.

Also provided is a composition comprising (1) a target polynucleotide; (2) a first target-hybridizing probe comprises a target binding sequence (P1-DNA) which is complementary to a first sequence on a strand of the target polynucleotide and a probe-binding sequence (P1-P); (3) a second target-hybridizing probe comprises a target binding sequence (P2-DNA) which is complementary to a second sequence on the same strand of the target polynucleotide and a probe-binding sequence (P2-P); (4) a non-target-hybridizing probe 3 labeled with label A; and (5) a non-target-hybridizing probe 4 labeled with label B, where the P1-P sequence is complementary to probe 3 and the P2-P sequence is complementary to probe 4, and the label A interacts with the label B to generate a signal indicative of an amount of the target polynucleotide.

In one embodiment, the first target-hybridizing probe and the second target-hybridizing probe hybridize to a same strand of said target polynucleotide in close proximity.

In another aspect, it is provided a method for detecting the amount of a target polynucleotide in a sample, comprising: (a) providing a target-hybridizing probe 1 comprising a target binding sequence (P1-DNA) which hybridizes to one strand of the target polynucleotide and a probe binding sequence (P1-P) which does not hybridize to the target polynucleotide, and a target-hybridizing probe 2 comprising a target binding sequence (P2-DNA) which hybridizes, in close proximity, to the same strand of the target polynucleotide and a probe binding sequence (P2-P) which does not hybridize to the target polynucleotide; (b) providing a non-target-hybridizing universal probe 3 labeled with label A and a non-target-hybridizing universal probe 4 labeled with label B, where the universal probe 3 hybridizes to the P1-P sequence and the universal probe 4 hybridizes to the P2-P sequence, and where the label A interact with the label B to generate a detectable signal; and (c) detecting the generated signal which is indicative as to the amount of the polynucleotide in the sample.

The present disclosure also relates to a method for detecting the amount of a target polynucleotide in an amplification reaction mixture, comprising: (a) providing a forward and a reverse primer which amplify the target polynucleotide in the amplification reaction mixture; (b) providing to the reaction mixture a target-hybridizing probe 1 comprising a target binding sequence (P1-DNA) which hybridizes to one strand of the target polynucleotide and a probe binding sequence (P1-P) which does not hybridize to the target polynucleotide, and a target-hybridizing probe 2 comprising a target binding sequence (P2-DNA) which hybridizes, in close proximity, to the same strand of the target polynucleotide and a probe binding sequence (P2-P) which does not hybridize to the target polynucleotide; (c) providing to the reaction mixture a non-target-hybridizing universal probe 3 labeled with label A and a non-target-hybridizing universal probe 4 labeled with label B, where the universal probe 3 hybridize to the P1-P sequence and the universal probe 4 hybridizes to the P2-P sequence, and where the label A interact with the label B to generate a signal; and (d) detecting the generated signal which is indicative as to the amount of the polynucleotide in the sample.

Preferably, in the subject composition or method, the label A interacts with the label B to generate a signal indicating the amount of the target polynucleotide. Also preferably, the label A and label B may be members of a pair of interactive labels. More preferably, the label A and label B may be fluorescent dyes. In one embodiment, the label A and label B may be a donor-acceptor pair which interact with each other to generate a signal by fluorescent resonance energy transfer (FRET). In a preferred embodiment, the donor-acceptor pair may be a FAM/ROX pair. In another preferred embodiment, the acceptor may be a dark quencher.

Preferably, the probe 3 or 4 shares no homology to any polynucleotide isolated from the sample containing the target polynucleotide. In one embodiment, the probe 3 is labeled at its 3' terminal and the probe 4 is labeled at its 5' terminal. Preferably, the 3' terminal of a probe is modified to prevent probe extension. In one embodiment, the 3' terminal of a probe is phosphorylated to prevent probe extension. In one embodiment, the target binding sequence locates at 5' of the probe binding sequence in the probe 1, while the target binding sequence locates at 3' of the probe binding sequence in the probe 2. Preferably, the universal probe 3 hybridizes to the P1-P sequence and the universal probe 4 hybridizes to the P2-P sequence. In a preferred embodiment, the universal probe 3 has a higher melting point^{™} than the P1-DNA sequence, and the universal probe 4 has a higher Tm than the P2-DNA sequence. Preferably, the P1-DNA and P2-DNA binds to the strand amplified by the reverse primer, the amount of the forward primer to the amount of the reverse primer in the composition is 1:5; and when the P1-DNA and P2-DNA binds to the strand amplified by the forward primer and the amount of the forward primer to the amount of the reverse primer is 5:1.

In one embodiment, the subject composition further comprises a forward and a reverse primer used to amplify the target polynucleotide. In another embodiment, the subject composition further comprises a control polynucleotide. In one embodiment, the amplification reaction is a polymerase chain reaction (PCR). In another embodiment, the generated detectable signal is detected at the end of two or more PCR cycles.

Preferably, the generated signal is detected and compared with signals generated by one or more reference containing a known amount of the target polynucleotide for the determination of the amount of the target polynucleotide in the sample or in the amplification reaction mixture.

The disclosure also includes kits for the subject compositions. Kits are preferably packaged in a unit container and may contain the reagents in pre-measured amounts designed to operate with each other so as to produce the desired result. The kits may further comprise one or more of the following items, DNA polymerase, control probes, control target polynucleotide, reaction buffer, amplification primer, exonuclease for degrading excess amplification primer, and hybridization/washing buffer.

The present invention provides a composition comprising:
- a polynucleotide target;
- a first target-hybridizing probe which comprises a target binding sequence (P1-DNA) which hybridizes to a strand of said target polynucleotide and a probe binding sequence (P1-P);
- a second target-hybridizing probe which comprises a target binding sequence (P2-DNA) which hybridizes, in close proximity to said first target-hybridizing probe and which hybridizes to the same strand of said target polynucleotide as said first target-hybridizing probe and wherein the second target-hybridizing probe further comprises a probe binding sequence (P2-P);
- a non-target-hybridizing probe 3 labeled with label A; and
- a non-target-hybridizing probe 4 labeled with label B, wherein said probe 3 hybridizes to said P1-P sequence and said probe 4 hybridizes to said P2-P sequence and wherein said label A and label B are interactive labels.

The present invention also provides a composition as mentioned above, further characterized in that:
- said target binding sequence is at 5' of said probe binding in said probe 1, while said target binding sequence is at 3' of said probe binding sequence in said probe 2,
- said composition further comprises a forward and a reverse primer for the amplification of said target polynucleotide, and/or
- said composition further comprises a control polynucleotide.

The present invention also provides a composition as mentioned above, wherein when said P1-DNA and P2-DNA bind to the strand amplified by said reverse primer, the amount of said forward primer to the amount of said reverse primer in said composition is 1 : 5; and when said P1-DNA and P2-DNA binds to the strand amplified by said forward primer, the amount of said forward primer to the amount of said reverse primer is 5 : 1.

The present invention also provides a composition as mentioned above, further characterized in that:
- said label A and label B are fluorescent dyes,
- said label A and label B are a donor-acceptor pair which interact with each other to generate a signal by fluorescent resonance energy transfer (FRET),
- said probe 3 or 4 shares no homology to any polynucleotide isolated from a sample containing said target polynucleotide,
- said probe 3 is labeled at its 3' terminal and said probe 4 is labeled at its 5' terminal,
- the 3' terminal of a probe is modified to prevent probe extension,
- said 3' terminal of a probe is phosphorylated, and/or
- said probe 3 has a higher melting point (Tm) than said P1-DNA sequence, and said probe 4 has a higher Tm than said P2-DNA sequence.

The present invention also provides a composition as mentioned above, further characterized in that said donor-acceptor pair is a FAM/ROX pair, and/or said acceptor is a dark quencher.

The present invention further provides a kit comprising:
- a polynucleotide target;
- a first target-hybridizing probe which comprises a target binding sequence (P1-DNA) which hybridizes to a strand of said target polynucleotide and a probe binding sequence (P1-P);
- a second target-hybridizing probe which comprises a target binding sequence (P2-DNA) which hybridizes, in close proximity to said first target-hybridizing probe and which hybridizes to the same strand of said target polynucleotide as said first target-hybridizing probe and wherein the second target-hybridizing probe further comprises a probe binding sequence (P2-P) - a non-target-hybridizing probe 3 labeled with label A;
- a non-target-hybridizing probe 4 labeled with label B, wherein said probe 3 hybridizes to said P1-P sequence and said probe 4 hybridizes to said P2-P sequence and wherein said label A and label B are interactive labels; and
- packaging materials therefore.

The present invention also provides a kit as mentioned above, further characterized in that:
- the 3' terminal of a probe is modified to prevent probe extension,
- said 3' terminal of a probe is phosphorylated,
- said kit further comprises a forward and a reverse primer for the amplification of said target polynucleotide,
- said kit further comprises a control polynucleotide, and/or
- said target binding sequence is at 5' of said probe binding in said probe 1, while said target binding sequence is at 3' of said probe binding in said probe 2.

The present invention also provides a kit as mentioned above, wherein when said P1-DNA and P2-DNA binds to the strand amplified by said reverse primer, the amount of said forward primer to the amount of said reverse primer in said kit is 1 : 5; and when said P1-DNA and P2-DNA binds to the strand amplified by said forward primer, the amount of said forward primer to the amount of said reverse primer is 5 : 1.

The present invention also provides a kit as mentioned above, further characterized in that:
- said label A and label B are fluorescent dyes,
- said label A and label B are a donor-acceptor pair which interact with each other to generate a signal by fluorescent resonance energy transfer (FRET),
- said probe 3 or 4 shares no homology to any polynucleotide isolated from a sample containing said target polynucleotide,
- said probe 3 is labeled at its 3' terminal and said probe 4 is labeled at its 5' terminal, and/or
- said probe 3 has a higher melting point (Tm) than said P1-DNA sequence, and said probe 4 has a higher Tm than said P2-DNA sequence.

The present invention also provides a kit as mentioned above, further characterized in that: said donor-acceptor pair is a FAM/ROX pair, and/or said acceptor is a dark quencher.

Moreover, the present invention provides a method for detecting the amount of a target polynucleotide, comprising:
(a) adding to said target polynucleotide:
   (1) a target-hybridizing probe 1 comprising a target binding sequence (P1-DNA) which hybridizes to one strand of said target polynucleotide and a probe binding sequence (P1-P),
   (2) a target-hybridizing probe 2 comprising a target binding sequence (P2-DNA) which hybridizes, in close proximity to said first target-hybridizing probe and which hybridizes to the same strand of said target polynucleotide as said first target-hybridizing probe and a probe binding sequence (P2-P) ;
   (3) a non-target-hybridizing probe 3 labeled with label A which hybridizes to said P1-P sequence, and
   (4) a non-target-hybridizing probe 4 labeled with label B which hybridizes to said P2-P sequence, wherein said addition permits said label A to interact with said label B to generate a detectable signal; and
(b) detecting said generated signal as indicative of the amount of said polynucleotide.

In addition, the present invention provides a method for detecting the amount of a target polynucleotide in an amplification reaction mixture, comprising:
(a) adding to said amplification reaction mixture:
   (1) a target-hybridizing probe 1 comprising a target binding sequence (P1-DNA) which hybridizes to one strand of said target polynucleotide and a probe binding sequence (P1-P),
   (2) a target-hybridizing probe 2 comprising a target binding sequence (P2-DNA) which hybridizes, in close proximity to said first target-hybridizing probe and which hybridizes to the same strand of said target polynucleotide as said first target-hybridizing probe and a probe binding sequence (P2-P),
   (3) a non-target-hybridizing probe 3 labeled with label A which hybridizes to said P1-P sequence, and
   (4) a non-target-hybridizing probe 4 labeled with label B which hybridizes to said P2-P sequence, wherein said addition permits said label A to interact with said label B to generate a signal; and
(b) detecting said generated signal as indicative of the amount of said polynucleotide.

The present invention also provides a method as mentioned above, further characterized in that:
- when said P1-DNA and P2-DNA bind to the strand amplified by a reverse primer, the amount of a forward primer to the amount of said reverse primer in said reaction mixture is 1 : 5; and when said P1-DNA and P2-DNA bind to the strand amplified by a forward primer, the amount of said forward primer to the amount of a reverse primer is 5 : 1, and/or
- said amplification reaction is a polymerase chain reaction (PCR).

The present invention also provides a method as mentioned above, wherein said generated detectable signal is detected at the end of two or more PCR cycles.

The present invention also provides a method as mentioned above, further characterized in that:
- said label A and label B are fluorescent dyes,
- said label A and label B are a donor-acceptor pair which interact with each other to generate a signal by fluorescent resonance energy transfer (FRET),
- said probe 3 or 4 shares no homology to any polynucleotide isolated from a sample containing said target polynucleotide,
- said probe 3 is labeled at its 3' terminal and said probe 4 is labeled at its 5' terminal,
- the 3' terminal of a probe is modified to prevent probe extension,
- said 3' terminal of a probe is phosphorylated,
- said target binding sequence is at 5' of said probe binding in said probe1, while said target binding sequence is at 3' of said probe binding in said probe 2,
- said probe 3 has a higher melting point (Tm) than said P1-DNA sequence, and said probe 4 has a higher Tm than said P2-DNA sequence, and/or
- said generated signal is detected and compared with signals generated by one or more reference containing a known amount of said target polynucleotide for the determination of the amount of said target polynucleotide.

The present invention also provides a method as mentioned above, further characterized in that: said donor-acceptor pair is a FAM/ROX pair, and/or said acceptor is a dark quencher.

### BRIEF DESCRIPTION OF DRAWINGS

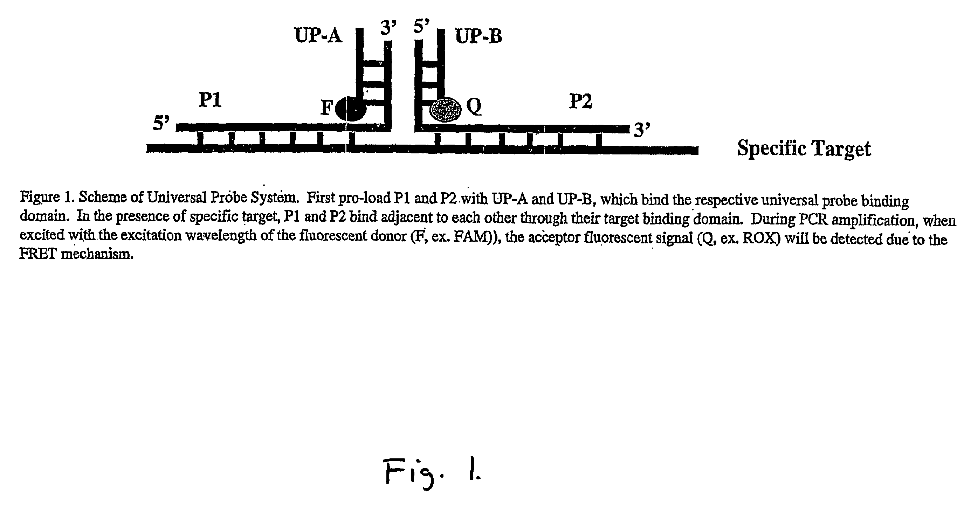

Figure 1 illustrates the scheme of a universal probe system according to one embodiment of the invention. In this embodiment, P1 and P2 (target-hybridizing probes) are first pre-loaded with UP-A and UP-B (non-target-hybridizing universal probes). The universal probes bind the respective universal probe binding sequences within the target-hybridizing probes. In the presence of specific target, P and P2 bind to each other in close proximity through their target binding sequences. During PCR amplification, when excited with the excitation wavelength of the fluorescent donor (e.g., FAM), the receptor fluorescent signal (e.g., ROX) will be detected due to the FRET mechanism.

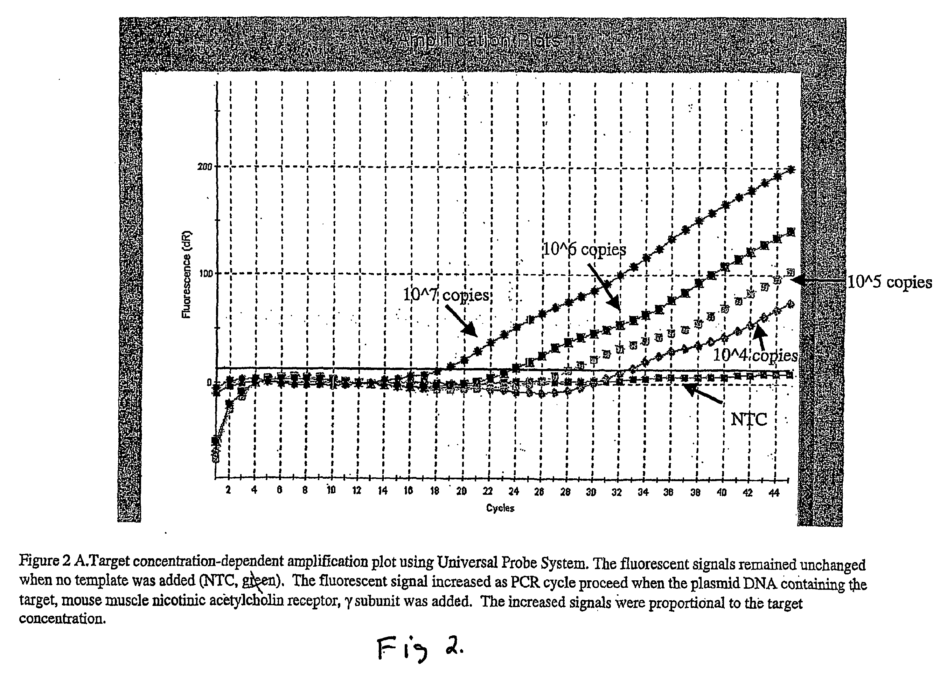

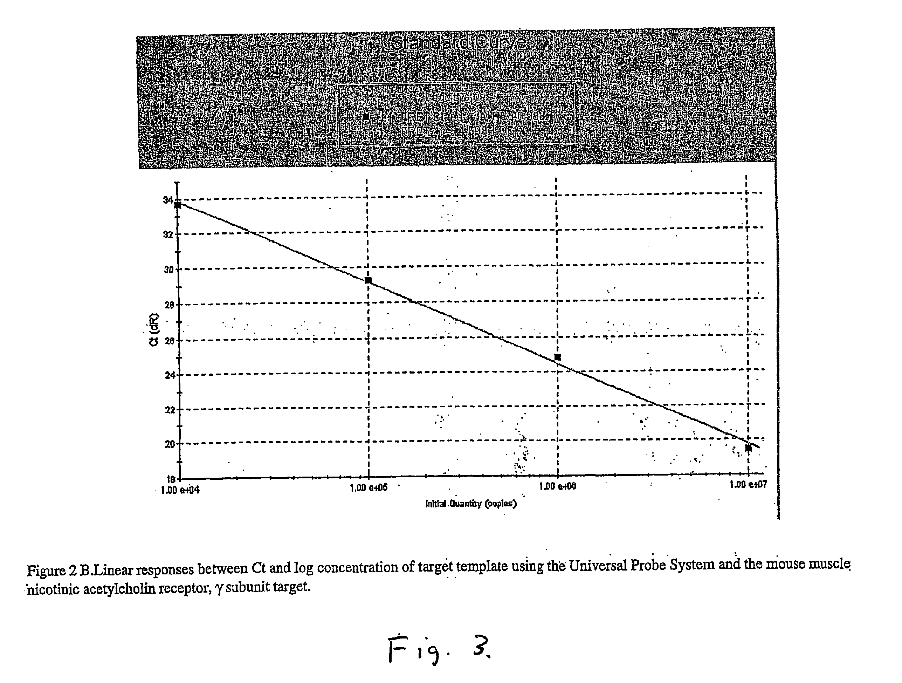

Figure 2A is a graph showing target concentration-dependent amplification plot using a universal probe system according to one embodiment of the invention. The fluorescent signals remained unchanged when no template was added (NTC). The fluorescent signal increased as PCR cycle proceed when the plasmid DNA containing the target, mouse muscle nicotinic acetylcholin receptor, γ subunit was added. The increased signals were proportional to the target concentration.
Figure 2B is a graph showing linear responses between Ct and log concentration of target template using a universal probe system and the mouse muscle nicotinic acetylcholin receptor, g subunit target, according to one embodiment of the invention.

### DETAILED DESCRIPTION OF THE INVENTION

### Definitions

As used herein, the term "target polynucleotide" refers to a polynucleotide whose amount is to be determined in a sample. A "target polynucleotide" of the present invention contains a known sequence of at least 20 nucleotides, preferably at least 50 nucleotides, more preferably at least 100 or more nucleotides, for example, 500 or more nucleotides. A "target polynucleotide" of the invention may be a naturally occurring polynucleotide (i.e., one existing in nature without human intervention), or a recombinant polynucleotide (i.e., one existing only with human intervention). The target polynucleotide also includes amplified products of itself, for example, as in a polymerase chain reaction. According to the invention, a "target polynucleotide" may contain a modified nucleotide which include phosphorothioate, phosphite, ring atom modified derivatives, and the like.

As used herein, a "polynucleotide" refers to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next. The term "polynucleotide" includes, without limitation, single- and double-stranded polynucleotide. The term "polynucleotide" as it is employed herein embraces chemically, enzymatically or metabolically modified forms of polynucleotide. "Polynucleotide" also embraces a short polynucleotide, often referred to as an oligonucleotide (e.g., a primer or a probe). A polynucleotide has a "5'-terminus" and a "3'-terminus" because polynucleotide phosphodiester linkages occur to the 5' carbon and 3' carbon of the pentose ring of the substituent mononucleotides. The end of a polynucleotide at which a new linkage would be to a 5' carbon is its 5' terminal nucleotide. The end of a polynucleotide at which a new linkage would be to a 3' carbon is its 3' terminal nucleotide. A terminal nucleotide, as used herein, is the nucleotide at the end position of the 3'- or 5'-terminus. As used herein, a polynucleotide sequence, even if internal to a larger polynucleotide (e.g., a sequence region within a polynucleotide), also can be said to have 5'- and 3'- ends.

As used herein, the term "oligonucleotide" refers to a short polynucleotide, typically less than 150 nucleotides long (e.g., between 5 and 150, preferably between 10 to 100, more preferably between 15 to 50 nucleotides in length). However, as used herein, the term is also intended to encompass longer or shorter polynucleotide chains. An "oligonucleotide" may hybridize to other polynucleotides, therefore serving as a probe for polynucleotide detection, or a primer for polynucleotide chain extension.

As used herein, a "primer" refers to a type of oligonucleotide having or containing the length limits of an "oligonucleotide" as defined above, and having or containing a sequence complementary to a target polynucleotide, which hybridizes to the target polynucleotide through base pairing so to initiate an elongation (extension) reaction to incorporate a nucleotide into the oligonucleotide primer. The length of a primer is the same as generally described herein above for an oligonucleotide.

As used herein, a "probe" refers to a type of oligonucleotide having or containing a sequence which is complementary to another polynucleotide, e.g., a target polynucleotide or another oligonucleotide. A "probe" according to the invention, may be between 10 to 100 nucleotides in length, and between 15-50 nucleotides in length within the definition of probe.

As used herein, a "target-hybridizing probe," refers to a probe which hybridizes to a target polynucleotide. In one embodiment, a "target-hybridizing probe" of the present invention. includes a first sequence (a target binding sequence : "P-DNA") which hybridizes to the target polynucleotide and a second sequence (a universal probe binding sequence: "P-P") which binds to a probe, e.g., a non-target-hybridizing probe, but does not hybridize to the target polynucleotide.

A "non-target-hybridizing probe," refers to a probe which hybridizes to a "target-hybridizing probe", but not to the target polynucleotide itself. In one embodiment, a "non-target-hybridizing probe" of the present invention includes a sequence which hybridizes to the second sequence within the target-hybridizing probe. A common nucleotide sequence may be shared by the second sequence of two or more target-hybridizing probes, so that a common non-target-hybridizing probe is used to hybridize to two or more target-hybridizing probes and this probe does not hybridize to a target. The second sequence may be independent of the target polynucleotide sequence, i.e., the second sequence is commonly shared by two or more target-hybridizing probes which are used to hybridize with different target polynucleotides. Such a non-target-hybridizing probe which hybridizes to a common and independent second sequence is referred to as a "universal probe." The first sequence or the second sequence of a probe may be between 10 and 100 nucleotides in length, between 15-50 nucleotides in length, and between 15-30 nucleotides in length.

As used herein, the term "in close proximity," refers to the relative distance to which two target-hybridizing probes hybridize to the same strand of a target polynucleotide, the distance being sufficient to permit the interaction of labels on the two non-target-hybridizing probes which hybridize to the target-hybridizing probes. The distance between the two hybridization sites is less than 50 nucleotides, preferably less than 30 nucleotides, more preferably less than 10 nucleotides, for example, less than 6 nucleotides.

As used herein, the term "complementary" refers to the concept of sequence complementarity between regions of two polynucleotide strands or between two regions of the same polynucleotide strand. It is known that an adenine base of a first polynucleotide region is capable of forming specific hydrogen bonds ("base pairing") with a base of a second polynucleotide region which is antiparallel to the first region if the base is thymine or uracil. Similarly, it is known that a cytosine base of a first polynucleotide strand is capable of base pairing with a base of a second polynucleotide strand which is antiparallel to the first strand if the base is guanine. A first region of a polynucleotide is complementary to a second region of the same or a different polynucleotide if, when the two regions are arranged in an antiparallel fashion, at least one nucleotide of the first region is capable of base pairing with a base of the second region. Therefore, it is not required for two complementary polynucleotides to base pair at every nucleotide position. "Complementary" refers to a first polynucleotide that is 100% complementary to a second polynucleotide forms base pair at every nucleotide position. "Complementary" also refers to a first polynucleotide that is not 100% complementary (e.g., 90%, or 80% or 70% complementary) contains mismatched nucleotides at one or more nucleotide positions. In one embodiment, two complementary polynucleotides are capable of hybridizing to each other under high stringency hybridization conditions.

As used herein, the term "sample" refers to a biological material which is isolated from its natural environment and containing a polynucleotide. A "sample" according to the invention may consist of purified or isolated polynucleotide, or it may comprise a biological sample such as a tissue sample, a biological fluid sample, or a cell sample comprising a polynucleotide. A biological fluid includes blood, plasma, sputum, urine, cerebrospinal fluid, lavages, and leukophoresis samples. A sample of the present invention may be a plant, animal, bacterial or viral material containing a target polynucleotide. Useful samples of the present invention may be obtained from different sources, including, for example, but not limited to, from different individuals, different developmental stages of the same or different individuals, different disease individuals, normal individuals, different disease stages of the same or different individuals, individuals subjected to different disease treatment, individuals subjected to different environmental factors, individuals with predisposition to a pathology, individuals with exposure to an infectious disease (e.g., HIV). Useful samples may also be obtained from in vitro cultured tissues, cells, or other polynucleotide containing sources. The cultured samples may be taken from sources including, but are not limited to, cultures (e.g., tissue or cells) cultured in different media and conditions (e.g., pH, pressure, or temperature), cultures (e.g., tissue or cells) cultured for different period of length, cultures (e.g., tissue or cells) treated with different factors or reagents (e.g., a drug candidate, or a modulator), or cultures of different types of tissue or cells.

As used herein, a polynucleotide "isolated" from a sample is a naturally occurring polynucleotide sequence within that sample which has been removed from its normal cellular (e.g., chromosomal) environment. Thus, an "isolated" polynucleotide may be in a cell-free solution or placed in a different cellular environment.

As used herein, the term "amount" refers to an amount of a target polynucleotide in a sample, e.g., measured in µg, µmol or copy number. The abundance of a polynucleotide in the present invention is measured by the fluorescence intensity emitted by such polynucleotide, and compared with the fluorescence intensity emitted by a reference polynucleotide, i.e., a polynucleotide with a known amount.

As used herein, the term "homology" refers to the optimal alignment of sequences (either nucleotides or amino acids), which may be conducted by computerized implementations of algorithms. "Homology", with regard to polynucleotides, for example, may be determined by analysis with BLASTN version 2.0 using the default parameters. A "probe which shares no homology with another polynucleotide" refers to that the homology between the probe and the polynucleotide, as measured by BLASTN version 2.0 using the default parameters, is no more than 40%, e.g., less than 35%, or less than 30%, or leas than 25%, or less than 20%.

As used herein, a "detectable label" or a "label" refers to a molecule capable of generating a detectable signal, either by itself or through the interaction with another label. A " label" may be a directly detectable label or may be a member of a signal generating system, and thus can generate a detectable signal in context with other members of the signal generating system, e.g., a biotin-avidin signal generation system, or a donor-acceptor pair for fluorescent resonance energy transfer (FRET) (Stryer et al., 1978, Ann. Rev. Biochem., 47:819; Selvin, 1995, Methods Enzymol., 246:300). The label can be isotopic or non-isotopic, usually non-isotopic, and can be a catalyst, such as an enzyme (also referred to as an enzyme label), a polynucleotide coding for a catalyst, promoter, dye, fluorescent molecule (also referred to as a fluorescent label), chemiluminescer (also referred to as a chemiluminescent label), coenzyme, enzyme substrate, radioactive group (also referred to as a radiolabel), a small organic molecule, amplifiable polynucleotide sequence, a particle such as latex or carbon particle, metal sol, crystallite, liposome, cell, etc., which may or may not be further labeled with a dye (also referred to as a colorimetric label), catalyst or other detectable group, and the like. Preferably, a label of the present invention, is a member of a pair of interactive labels. The members of a pair of "interactive labels" interact and generate a detectable signal when brought in close proximity. The signals generated is preferably detectable by visual examination methods well known in the art, preferably by FRET assay. The members of a pair of interactive labels may be a donor and an acceptor, or a receptor and a quencher.

As used herein, the term "donor" refers to a fluorophore which absorbs at a first wavelength and emits at a second, longer wavelength. The term "acceptor" refers to a fluorophore, chromophore or quencher with an absorption spectrum which overlaps the donor's emission spectrum and is able to absorb some or most of the emitted energy from the donor when it is near the donor group (typically between 1-100nm). If the acceptor is a fluorophore capable of exhibiting FRET, it then re-emits at a third, still longer wavelength; if it is a chromophore or quencher, then it releases the energy absorbed from the donor without emitting a photon. Although the acceptor's absorption spectrum overlaps the donor's emission spectrum when the two groups are in proximity, this need not be the case for the spectra of the molecules when free in solution. Acceptors thus include fluorophores, chromophores or quenchers that, following attachment to either a chain terminator or to an anti-tag molecule, show alterations in absorption spectrum which permit the group to exhibit either FRET or quenching when placed in proximity to the donor through the binding interactions of the anti-tag molecule and a tag molecule comprising the chain terminator.

As used herein, references to "fluorescence" or "fluorescent groups" or "fluorophores" include luminescence and luminescent groups, respectively.

As used herein, the term "hybridization" is used in reference to the pairing of complementary (including partially complementary) polynucleotide strands. Hybridization and the strength of hybridization (i.e., the strength of the association between polynucleotide strands) is impacted by many factors well known in the art including the degree of complementarity between the polynucleotides, stringency of the conditions involved affected by such conditions as the concentration of salts, the melting temperature (Tm) of the formed hybrid, the presence of other components (e.g., the presence or absence of polyethylene glycol), the molarity of the hybridizing strands and the G:C content of the polynucleotide strands.

As used herein, when one polynucleotide is said to "hybridize" to another polynucleotide, it means that there is some complementarity between the two polynucleotides or that the two polynucleotides form a hybrid at a high stringency condition. When one polynucleotide is said to not hybridize to another polynucleotide, it means that there is no sequence complementarity between the two polynucleotides or that no hybrid forms between the two polynucleotides at a high stringency condition.

As used herein, the term "stringency" is used in reference to the conditions of temperature, ionic strength, and the presence of other compounds, under which polynucleotide hybridization is conducted. With "high stringency" conditions, polynucleotide pairing will occur only between polynucleotide fragments that have a high frequency of complementary base sequences. Thus, conditions of "weak" or "low" stringency are often required when it is desired that polynucleotides which are not completely complementary to one another be hybridized or annealed together. The art knows well that numerous equivalent conditions can be employed to comprise high or low stringency conditions.

As used herein, "high stringency conditions" refer to temperature and ionic condition used during polynucleotide hybridization and/or washing. The extent of "high stringency" is nucleotide sequence dependent and also depends upon the various components present during hybridization. Generally, highly stringent conditions are selected to be about 5 to 20 degrees C lower than the thermal melting point (Tm) for the specific sequence at a defined ionic strength and pH. "High stringency conditions", as used herein, refer to a washing procedure including the incubation of two or more hybridized polynucleotides in an aqueous solution containing 0.1X SSC and 0.2% SDS, at room temperature for 2-60 minutes, followed by incubation in a solution containing 0.1X SSC at room temperature for 2-60 minutes. "High stringency conditions" are known to those of skill in the art, and may be found in, for example, Maniatis et al., 1982, Molecular Cloning, Cold Spring Harbor Laboratory and Schena, ibid.

As used herein, "low stringency conditions" refer to a washing procedure including the incubation of two or more hybridized polynucleotides in an aqueous solution comprising IX SSC and 0.2% SDS at room temperature for 2 - 60 minutes.

As used herein, the term "Tm " is used in reference to the "melting temperature". The melting temperature is the temperature at which 50% of a population of double-stranded polynucleotide molecules becomes dissociated into single strands. The equation for calculating the Tm of polynucleotides is well-known in the art. For example, the Tm may be calculated by the following equation: Tₘ=69.3 + 0.41 X (G+C)% - 650/L, wherein L is the length of the probe in nucleotides. The Tm of a hybrid polynucleotide may also be estimated using a formula adopted from hybridization assays in 1 M salt, and commonly used for calculating Tm for PCR primers: [(number of A+T) x 2°C + (number of G+C) x 4°C], see, for example, C. R. Newton et al. PCR, 2nd Ed., Springer-Verlag (New York: 1997), p. 24. Other more sophisticated computations exist in the art which take structural as well as sequence characteristics into account for the calculation of Tm. A calculated Tm is merely an estimate; the optimum temperature is commonly determined empirically.

"Primer extension reaction" or "chain elongation reaction" means a reaction between a target-primer hybrid and a nucleotide which results in the addition of the nucleotide to a 3'-end of the primer such that the incorporated nucleotide is complementary to the corresponding nucleotide of the target polynucleotide. Primer extension reagents typically include (i) a polymerase enzyme; (ii) a buffer; and (iii) one or more extendible nucleotides.

As used herein, "polymerase chain reaction" or "PCR" refers to an in vitro method for amplifying a specific polynucleotide template sequence. The PCR reaction involves a repetitive series of temperature cycles and is typically performed in a volume of 50-100 µl. The reaction mix comprises dNTPs (each of the four deoxynucleotides dATP, dCTP, dGTP, and dTTP), primers, buffers, DNA polymerase, and polynucleotide template. One PCR reaction may consist of 5 to 100 "cycles" of denaturation and synthesis of a polynucleotide molecule.

As used herein, "polynucleotide polymerase" refers to an enzyme that catalyzes the polymerization of nucleotide. Generally, the enzyme will initiate synthesis at the 3'-end of the primer annealed to a polynucleotide template sequence, and will proceed toward the 5' terminal of the template strand. "DNA polymerase" catalyzes the polymerization of deoxynucleotides. Useful DNA polymerases include, but are not limited to, exo⁺ DNA polymerases, for example, Pyrococcus furiosus (Pfu) DNA polymerase (Lundberg et al., 1991, Gene, 108:1; U.S. Patent No. 5,556,772, Thermus thermophilus (Tth) DNA polymerase (Myers and Gelfand 1991, Biochemistry 30:7661), Bacillus stearothermophilus DNA polymerase (Stenesh and McGowan, 1977, Biochim Biophys Acta 475:32), Thermococcus litoralis (Tli) DNA polymerase (also referred to as Vent DNA polymerase, Cariello et al., 1991, Polynucleotides Res, 19: 4193), Thermotoga maritima (Tma) DNA polymerase (Diaz and Sabino, 1998 Braz J. Med. Res, 31:1239), Pyrococcus kodakaraensis KOD DNA polymerase (Takagi et al., 1997, Appl. Environ. Microbiol. 63:4504), JDF-3 DNA polymerase (Patent application WO 0132887), and Pyrococcus GB-D (PGB-D) DNA polymerase (Juncosa-Ginesta et al., 1994, Biotechniques, 16:820). Useful DNA polymerases also include exo⁻ DNA polymerases, for example, exo- Pfu DNA polymerase (a mutant form of Pfu DNA polymerase that substantially lacks 3' to 5' exonuclease activity, Cline et al., 1996, Nucleic Acids Research, 24: 3546; US Patent No. 5,556,772; commercially available from Stratagene, La Jolla, Calif. Catalogue # 600163), exo- Tma DNA polymerase (a mutant form of Tma DNA polymerase that substantially lacks 3' to 5' exonuclease activity), exo- Tli DNA polymerase (a mutant form of Tli DNA polymerase that substantially lacks 3' to 5' exonuclease activity New England Biolabs, (Cat #257)), exo- E. coli DNA polymerase (a mutant form of E. coli DNA polymerase that substantially lacks 3' to 5' exonuclease activity) exo-klenow fragment of E.col; DNA polymerase I (Stratagene, Cat #600069), exo- T7 DNA polymerase (a mutant form of T7 DNA polymerase that substantially lacks 3' to 5' exonuclease activity), exo- KOD DNA polymerase (a mutant form of KOD DNA polymerase that substantially lacks 3' to 5' exonuclease activity), exo- JDF-3 DNA polymerase (a mutant form of JDF-3 DNA polymerase that substantially lacks 3' to 5' exonuclease activity), exo- PGB-D DNA polymerase (a mutant form of PGB-D DNA polymerase that substantially lacks 3' to 5' exonuclease activity) New England Biolabs, Cat. #259, Tth DNA polymerase, Taq DNA polymerase (e.g., Cat. Nos 600131, 600132, 600139, Stratagene La Jolla, Calif.); UlTma (N-truncated) Thermatoga martima DNA polymerase; Klenow fragment of DNA polymerase I, 9°Nm DNA polymerase (discontinued product from New England Biolab, Beverly, MA), and "3'-5' exo reduced" mutant (Southworth et al., 1996, Proc. Natl. Acad. Sci 93:5281). The polymerase activity of any of the above enzyme can be defined by means well known in the art. One unit of DNA polymerase activity, according to the subject invention, is defined as the amount of enzyme which catalyzes the incorporation of 10 nmoles of total dNTPs into polymeric form in 30 minutes at optimal temperature.

"Nucleotide Analog" refers to a nucleotide in which the pentose sugar and/or one or more of the phosphate esters is replaced with its respective analog. Exemplary pentose sugar analogs are those previously described in conjunction with nucleoside analogs. Exemplary phosphate ester analogs include, but are not limited to, alkylphosphonates, methylphosphonates, phosphoramidates, phosphotriesters, phosphorothioates, phosphorodithioates, phosphoroselenoates, phosphorodiselenoates, phosphoroanilothioates, phosphoroanilidates, phosphoroamidates, boronophosphates, etc., including any associated counterions, if present. Also included within the definition of "nucleotide analog" are nucleobase monomers which can be polymerized into polynucleotide analogs in which the DNA/RNA phosphate ester and/or sugar phosphate ester backbone is replaced with a different type of linkage.

As used herein, the term "opposite orientation", when refers to primers, means that one primer (i.e., the reverse primer) comprises a nucleotide sequence complementary to the sense strand of a target nucleic acid template, and another primer (i.e., the forward primer) comprises a nucleotide sequence complementary to the antisense strand of the same target nucleic acid template. Primers with opposite orientations may generate a PCR amplified product from matched nucleic acid template to which they complement.

As used herein, the term "same orientation", means that both or all primers comprise nucleotide sequences complementary to the same strand of a target nucleic acid template. Primers with same orientation will not generate a PCR amplified product from matched nucleic acid template to which they complement.

### Description

The present disclosure relates to a universal probe system for polynucleotide detection and is based on the use of one or more target-hybridizing and non-target-hybridizing probes, as well as a signal generated through the interaction of the non-target-hybridizing probes.

The universal probe system of the present invention may be used to monitor a polynucleotide amplification process, e.g. such as in real-time PCR, or it may be used for the detection of any polynucleotide not amplified.

When the universal probes are used for detecting the presence or amount of a target polynucleotide in a sample, the present disclosure relates to a target-hybridizing probe 1 comprising a target binding sequence (P1-DNA) which hybridizes to one strand of the target polynucleotide and a probe binding sequence (P1-P) which does not hybridize to the target polynucleotide, and a target-hybridizing probe 2 comprising a target binding sequence (P2-DNA) which hybridizes, in close proximity, to the same strand of the target polynucleotide and a probe binding sequence (P2-P) which does not hybridize to the target polynucleotide.

In one embodiment, a non-target-hybridizing universal probe 3 labeled with label A hybridizes to the P1-P sequence, and a non-target-hybridizing universal probe 4 labeled with label B hybridizes to the P2-P sequence. After the two universal probes hybridize to the two target-hybridizing probes, label A and label B are brought into close proximity, and the interaction between label A and label B generates a detectable signal which indicates the amount of the target polynucleotide in the sample.

When the universal probe system of the present invention is used to monitor the amplification process of an amplification reaction, the two target-hybridizing probes and the two universal probes are added into the amplification reaction mixture, either before the addition of amplification primers or after the addition of amplification primers, but preferably before the addition of DNA polymerase. The amplification of a target polynucleotide may be monitored during the process of amplification, for example, during or at the end of each cycle of a PCR reaction.

### Preparation of Primers and Probes

Probes and primers are typically prepared by biological or chemical synthesis, although they can also be prepared by biological purification or degradation, e.g., endonuclease digestion.

For short sequences such as probes and primers used in the present invention, chemical synthesis is frequently more economical as compared to biological synthesis. For longer sequences standard replication methods employed in molecular biology can be used such as the use of M13 for single stranded DNA as described by Messing, 1983, Methods Enzymol. 101: 20 - 78. Chemical methods of polynucleotide or oligonucleotide synthesis include phosphotriester and phosphodiester methods (Narang, et al., Meth. Enzymol. (1979) 68:90) and synthesis on a support (Beaucage, et al., Tetrahedron Letters. (1981) 22:1859 -1862) as well as phosphoramidate technique, Caruthers, M. H., et al., Methods in Enzymology (1988)154:287 - 314 (1988), and others described in "Synthesis and Applications of DNA and RNA," S. A. Narang, editor, Academic Press, New York, 1987, and the references contained therein.

Oligonucleotide probes and primers can be synthesized by any method described above and other methods known in the art.

In one embodiment, the method for detecting a target polynucleotide of the present invention involves the use of two target-hybridizing probes and two universal probes. Each of the two universal probes hybridize to one target-hybridizing probe.

In another embodiment, the method of the present invention involves the detection of two or more target polynucleotides, e.g., in a multiplex manner. In this case, there will be two target-hybridizing probes and two corresponding non-target-hybridizing probes for each target polynucleotide to be detected. The two corresponding non-target-hybridizing probes should only hybridize to the target-hybridizing probes used for the detection of the same target polynucleotide. In addition, the non-target-hybridizing probes for the detection of one target polynucleotide should be labeled with different labels so that the interaction between the labeled probes generate a distinguishable detectable signal from two other non-target-hybridizing probes used for the detection of another target polynucleotide.

The target-hybridizing probe of the present invention is designed to have a target binding sequence which hybridizes to the target polynucleotide and a probe binding sequence which hybridize to a universal probe. The target binding sequence, which hybridizes to the target polynucleotide, may have a sequence that is at least 70% (e.g., at least 80% or at least 90% or more) complementary to the target polynucleotide and comprises 10 to 100 nucleotides in length, preferably 15 to 50 nucleotides in length, more preferably 17-30 nucleotides in length. The probe binding sequence may be any sequence so long as it does not hybridize to the target polynucleotide and does not interfere with the hybridization of the target binding sequence to the target polynucleotide. Preferably, the probe binding sequence may be less than 30% (e.g., less than 20% or 10% or 5%) complementary to the target polynucleotide and comprises 10 to 50 nucleotides in length, preferably 15-30 nucleotides in length.

The two target-hybridizing probes used for the detection of the same target polynucleotide preferably have their probe binding sequence at different ends of the probes. For example, if one target-hybridizing probe (e.g., probe 1) has the probe binding sequence located 5' of the target binding sequence, the other target-hybridizing probe (e.g., probe 2) has the probe binding sequence located 3' of the target binding sequence, or vice versa.

The probe binding sequence, as well as the universal probe which hybridizes to it has a sequence that does not hybridize with the target polynucleotide and to have minimal homology to a polynucleotide from the same sample containing the target polynucleotide. For example, if the sample is a human sample, then the probe binding sequence or the universal probe is designed to have no homology to any human polynucleotides, e.g., DNAs or cDNAs isolated from human.

The probe binding sequence of the target-hybridizing probe, as well as the non-target-hybridizing probe of the present invention, preferably has a higher Tm (e.g., at least 2°C, or 4°C, or 6°C, or 8°C, or 10°C, or 15°C, or 20°C, or higher) than the respective target binding sequence of the target-hybridizing probe. In one embodiment, the target-hybridizing probes are pre-loaded with the universal sequences before hybridizing to the target polynucleotide molecules in a sample.

Preferably, the 3' terminal of a probe (e.g., a target-hybridizing probe or a universal probe) is blocked by adding a phosphate or an amine group, or the like to prevent chain elongation from the 3' terminal of the probe.

In a preferred embodiment, the probe binding sequence, according to the invention, may be a universal sequence (i.e., a common sequence) which is identical for a number of target-hybridizing probes which contain different target binding sequences. Therefore, each of the number of target-hybridizing probes also includes its unique target binding sequence which hybridizes to its unique target polynucleotide. The universal probe binding sequence does not hybridize to the target polynucleotide, it serves to provide a common sequence from which a universal non-target-hybridizing probe is based. The use of the universal probe binding sequence and the universal non-target-hybridizing probe for a number of different target-hybridizing probes, therefore, for the detection of a number of different target polynucleotides, avoids the laborious and costly design of a labeled specific oligonucleotide probe for each polynucleotide to be detected.

The universal probe of the present invention is preferably labeled. The two universal probes used for the detection of the same target polynucleotide are preferably labeled at different ends, that is, one universal probe (e.g., probe 3) is labeled at 3' end and the other universal probe (e.g., probe 4) is labeled at 5' end or vice versa. The labels on the two universal probes are preferably members of a pair of interactive labels, more preferably a donor-acceptor pair or a donor-quencher pair which can generate a detectable signal by FRET.

Nucleotide Analogs may be used in the universal probe for the purpose of labeling.

In a preferred embodiment of the invention, a conventional deoxynucleotide on a universal probe, e.g., the 3' or 5' terminal nucleotide, is labeled with a member of a pair of interactive labels. Non-limiting examples of some useful labeled nucleotide are listed in Table 1.

**Table 1. Examples of labeled nucleotides**

| **Fluorescein Labeled** | **Fluorophore Labeled** |
|---|---|
| Fluorescein - 12 - dCTP | Eosin - 6 - dCTP |
| Fluorescein - 12 - dUTP | Coumarin - 5 -ddUTP |
| Fluorescein - 12 - dATP | Tetramethylrhodamine - 6 - dUTP |
| Fluorescein - 12 - dGTP | Texas Red - 5 - dATP |
| Fluorescein - N6 - dATP | LISSAMINETM - rhodamine - 5 - dGTP |

| **FAM Labeled** | **TAMRA Labeled** |
|---|---|
| FAM - dUTP | TAMRA - dUTP |
| FAM - dCTP | TAMRA - dCTP |
| FAM-dATP | TAMRA-dATP |
| FAM - dGTP | TAMRA - dGTP |

| **ROX Labeled** | **JOE Labeled** |
|---|---|
| ROX - dUTP | JOE - dUTP |
| ROX - dCTP | JOE - dCTP |
| ROX - dATP | JOE - dATP |
| ROX - dGTP | JOE - dGTP |

| **R6G Labeled** | **R110 Labeled** |
|---|---|
| R6G - dUTP | R110 - dUTP |
| R6G - dCTP | R110-dCTP |
| R6G - dATP | R110 - dATP |
| R6G - dGTP | R110 - dGTP |

| **BIOTIN Labeled** | **DNP Labeled** |
|---|---|
| Biotin - N6 - dATP | DNP - N6 - dATP |

### Fluorescent Dyes

In a preferred embodiment, the universal probe of the present invention is labeled with a fluorescent dye. More preferably, the universal probe is labeled with a member of a pair of interactive labels.

Fluorescent dye-labeled polynucleotide or probes can be purchased from commercial sources. Labeled polynucleotides probes can also be prepared by any of a number of approaches. For example, labeling of the polynucleotide probe with a fluorescent dye can be done internally or by end labeling using methods well known in the art (see, for example, Ju et al., Proc Nat Acad Sci 92:4347-4351, 1995; Nelson et al. Polynucleotides Res 20:6253-6259, 1992.

Preferably, an oligonucleotide probe is labeled with a fluorescent dye. Fluorescent dyes useful as detectable labels are well known to those skilled in the art and numerous examples can be found in the Handbook of Fluoresdent Probes and Research Chemicals 6th Edition, Richard Haugland, Molecular Probes, Inc., 1996 (ISBN 0-9652240-0-7). The detectable label may be joined directly to the probe, or it may be joined through a linker. Examples of suitable linkers are described in U.S. Patent No. 5,770,716. The labels may be any fluorescent label or fluorophore that does not interfere with the ability of the oligonucleotide probe to hybridize to another polynucleotide (e.g., a target molecule or another probe). In a preferred embodiment, the two universal probes used for detecting the same target polynucleotide are labeled with any fluorescent labels or fluorophores which permit fluorescence resonance energy transfer. Detectable labels may be compounds or elements detectable by techniques other than, or in addition to, fluorescence. Such additional labels include radioisotopes, chemiluminescent compounds, spin labels, immunologically detectable haptens, and the like.

Preferably, fluorescent dyes are selected for compatibility with detection on an automated DNA sequencer and thus should be spectrally resolvable and not significantly interfere with electrophoretic analysis. Examples of suitable fluorescent dyes for use as detectable labels can be found in among other places, U.S. Patent Nos. 5,750,409; 5,366,860; 5,231,191; 5,840,999; 5,847,162; 4,439,356; 4,481,136; 5,188,934; 5,654,442; 5,840,999; 5,750,409; 5,066,580; 5,750,409; 5,366,860; 5,231,191; 5,840,999; 5,847,162; 5,486,616; 5,569,587; 5,569,766; 5,627;027; 5,321,130; 5,410,030; 5,436,134; 5,534,416; 5,582,977; 5,658,751; 5,656,449; 5,863,753; PCT Publications WO 97/36960; 99/27020; 99/16832; European Patent EP 0 050 684; Sauer et al,1995, J. Fluorescence 5:247-261; Lee et al., 1992, Nucl. Acids Res.20:2471-2483; mid Tu et al., 1998, Nucl. Acids Res. 26:2797-2802.

The oligonucleotide probe may be fluorescently labeled at any suitable position. Preferably, the fluorescent group is placed on or adjacent to the 5' or 3' terminal of the oligonucleotide probe.

Alternatively, the fluorescent group may be placed on or adjacent to the 3' or 5' end of a nucleotide within the oligonucleotide probe, for instance by incorporation of a fluorescent nucleotide derivative, modification of a nucleotide or substitution of a nucleotide by a fluorescent molecule. For example, tetramethylrhodamine (TAMRA) can be introduced into the oligonucleotide probe by incorporating the modified deoxy - thymidine phosphoramidite (5' - Dimethoxytrityloxy - 5 - [N - ((tetramethyl - odaminyl) - aminohexyl) - 3 - acryimido] - 2' - deoxy- thymidine - 3' - [(2 - cyanoethyl) - (N,N - diisopropyl)] - phosphoramidite). Fluorescein may be incorporated in an analogous way with: 5' - Dimethoxytrityloxy - 5 - [N - ((3',6' - dipivaloylfluoresceinyl) - aminohexyl) - 3 - acryimido] - 2' - deoxy - thymidine - 3' - [(2 - cyanoethyl) - (N,N - diisopropyl)] phosphoramidite. The DABCYL group may also be incorporated using 5' - Dimethoxytrityloxy - 5 - [N - ((4 - (dimethylamino)azobenzene) - aminohexyl) - 3 - acryimido] - 2' - deoxy - thymidine - 3' - [(2 - cyanoethyl) - (N,N - diisopropyl)] - phosphoramidite. More generally, a free amino group may be reacted with the active ester of any dye; such an amino group may be introduced by the inclusion of the modified thymidine 5' - Dimethoxytrityl- 5 - [N - (trifluoroacetylaminohexyl) - 3 - acrylimido]- 2'-deoxy - thymidine, 3' - [(2 - cyanoethyl) - (N,N - diisopropyl)] - phosphoramidite. Preferably, the incorporation of a modified base allows for normal base pairing. One skilled in the art should understand that thymidine in the above analogs may be substituted with other nucleotide (e.g., Guanosine, Adenosine, or Cytidine).

The oligonucleotide probes contain primary and secondary amines, hydroxyl, nitro and carbonyl groups. Methods that can be used to make fluorescent oligonucleotide probes and chain terminators are described below.

A number of chemical reactions can be applied to the fluorescent labeling of amines including but not limited to the following, where the fluorescent dye is conjugated to the indicated reactive group:

**Table 2**

| **Functional Group** | **Reaction** | **Product** |
|---|---|---|
| Amine | dye - isothiocyanates | Thiourea |
| Amine | dye - succinimidyl ester | Carboxamide |
| Amine | dye - sulfonyl chloride | Sulphonamide |
| Amine | dye - aldehyde | Alkylamine |

Oligonucleotide probes containing amine groups that are appropriate for the introduction of fluorescent dyes include but are not limited to those listed in Table 2.

A number of chemical reactions can be applied to the fluorescent labeling of ketone groups including but not limited to the following, where the fluorescent dye is conjugated to the indicated reactive group:

**Table 3**

| **Functional Group** | **Reaction** | **Product** |
|---|---|---|
| Ketone | dye - hydrazides | Hydrazones |
| Ketone | dye - semicarbazides | Hydrazones |
| Ketone | dye - carbohydrazides | Hydrazones |
| Ketone | dye - amines | Alkylamine |

Oligonucleotide probes containing ketone groups that are appropriate for the introduction of fluorescent dyes include but are not limited to those listed in Table 3.

A number of chemical reactions can be applied to the fluorescent labeling of aldehyde groups including but not limited to the following, where the fluorescent dye is conjugated to the indicated reactive group:

**Table 4**

| **Functional Group** | **Reaction** | **Product** |
|---|---|---|
| Aldehyde | dye - hydrazides | Hydrazones |
| Aldehyde | dye - semicarbazides | Hydrazones |
| Aldehyde | dye - carbohydrazides | Hydrazones |
| Aldehyde | dye - amines | Alkylamine |

Oligonucleotide probes containing aldehyde groups that are appropriate for the introduction of fluorescent dyes include but are not limited to those listed in Table 4.

Dehydrobutyrene and dehydroalanine moieties have characteristic reactions that can be utilized to introduce fluorophores, as illustrated but not limited to the following, where the fluorescent dye is conjugated to the indicated reactive group:

**Table 5**

| **Functional Group** | **Reaction** | **Product** |
|---|---|---|
| Dehydrobutyrine | dye - sulphydryl | Methyl lanthionine |
| Dehydroalanine | dye - sulphydryl | Lanthionine |

Oligonucleotide probes containing aldehyde groups that are appropriate for the introduction of fluorescent dyes include but are not limited to those listed in Table 5.

Other useful fluorophores (in addition to those listed in Tables 1-5) include, but are not limited to: Texas Red^{™} (TR), Lissamine^{™} rhodamine B, Oregon Green^{™} 488 (2',7' - difluorofluorescein), carboxyrhodol and carboxyrhodamine, Oregon Green^{™} 500, 6 - JOE (6 - carboxy - 4',5' - dichloro - 2',7' - dimethyoxyfluorescein, eosin F3S (6 - carobxymethylthio - 2',4', 5',7' - tetrabromo - trifluorofluorescein), cascade blue^{™} (CB), aminomethylcoumarin (AMC), pyrenes, dansyl chloride (5 - dimethylaminonaphthalene - 1 - sulfonyl chloride) and other napththalenes, PyMPO, ITC (1 - (3 - isothiocyanatophenyl) - 4 - (5 - (4 - methoxyphenyl)oxazol - 2 - yl)pyridinium bromide).

### Members of Pair Of Interactive Labels

A pair of interactive labels comprises a first and a second member. A first member may have more than one, e.g., two, three, or four different second members. The members may be a donor and an acceptor pair for generating detectable signal transfer. It is not critical which of the two universal probes for the detection of the same target polynucleotide is labeled with the donor or the acceptor. The stimulation of the acceptor by the donor, when brought to close proximity, generates a detectable signal. Different donor-acceptor pair generates different detectable signals which can be detected by FRET assay.

When two target-hybridizing probes hybridize to a target polynucleotide in close proximity, the two respective universal probes, either pre-loaded or hybridized to the target-hybridizing probes afterwards are brought to close proximity. This complex therefore brings the first and second members of a pair of interactive labels into proximity. When the members of the pair of interactive labels are donor-acceptor pair, the "fluorescence" of, or light emitted from, the complex is altered by fluorescence resonance energy transfer (FRET). "FRET" is a distance - dependent interaction between the electronic exited states of two dye molecules in which excitation is transferred from a donor molecule to an acceptor molecule. FRET is dependent on the inverse sixth power of the intermolecular separation, making it useful over distances comparable to the dimensions of biological macromolecules and obtainable in the complexes formed between the universal probes in the method of this invention. In some embodiments, the donor and acceptor dyes for FRET are different, in which case FRET can be detected by the appearance of sensitized fluorescence of the acceptor. When the donor and acceptor are the same, FRET is detected by the resulting fluorescence depolarization.

In one embodiment, the acceptor of the donor-acceptor pair is a dark quencher molecule, which emits heat instead of visible light as described below. In this case, the donor is also called a reporter.

The donor and acceptor groups may independently be selected from suitable fluorescent groups, chromophores and quenching groups. Donors and acceptors useful according to the invention include but are not limited to: 5 - FAM (also called 5 - carboxyfluorescein; also called Spiro (isobenzofuran - 1(3H), 9' - (9H)xanthene) - 5 - carboxylic acid,3',6' - dihydroxy - 3 - oxo - 6 - carboxyfluorescein); 5 - Hexachloro - Fluorescein ([4,7,2',4',5',7'- hexachloro - (3',6'-dipivaloyl- fluoresceinyl) - 6 - carboxylic acid]); 6 - Hexachloro - Fluorescein ([4,7,2',4',5',7' - hexachloro - (3',6' - dipivaloylfluoresceinyl) - 5 - carboxylic acid]); 5 - Tetrachloro - Fluorescein ([4,7,2',7' - tetra - chloro - (3',6' - dipivaloylfluoresceinyl) - 5 - carboxylic acid]); 6 - Tetrachloro- Fluorescein ([4,7,2',7' - tetrachloro - (3',6' - dipivaloylfluoresceinyl) - 6-carboxylic acid]); 5 - TAMRA (5 - carboxytetramethylrhodamine; Xanthylium, 9 - (2,4-dicarboxyphenyl) - 3,6 - bis(dimethyl- amino); 6 - TAMRA (6 - carboxytetramethylrhodamine; Xanthylium, 9 - (2,5 - dicarboxyphenyl) - 3, 6 - bis(dimethylamino); EDANS (5 - ((2 - aminoethyl) amino)naphthalene - 1 - sulfonic acid); 1,5 - IAEDANS (5 - ((((2 - iodoacetyl)amino)ethyl) amino)naphthalene - 1 - sulfonic acid); DABCYL (4 - ((4 - (dimethylamino)phenyl) azo)benzoic acid) Cy5 (Indodicarbocyanine - 5) Cy3 (Indo - dicarbocyanine - 3); and BODIPY FL (2,6 - dibromo - 4,4 - difluoro - 5,7 - dimethyl - 4 - bora - 3a,4a - diaza - s - indacene - 3 - proprionic acid), ROX, as well as suitable derivatives thereof.

Preferred combinations of donors and acceptors are listed as, but not limited to, the donor/acceptor pairs shown in Tables 6 and 7 (which includes values for Rₒ - the distance at which 50% of excited donors are deactivated by FRET).

**Table 6. Typical values of Rₒ**

| **Donor** | **Acceptor** | **Ro (Å)*** |
|---|---|---|
| Fluorescein | Tetramethylrhodamine | 55 |
| IAEDANS | Fluorescein | 46 |
| EDANS | DABCYL | 33 |
| Fluorescein | Fluorescein | 44 |
| BODIPY FL | BODIPY FL | 57 |

| | | |
|---|---|---|
| * Rₒ is the distance at which 50% of excited donors are deactivated by FRET. Data from Haugland, RP. 1996. Handbook of Fluorescent Probes and Research Chemicals, 6th edition. Molecular Probes, Inc. Eugene OR, USA. | | |

**Table 7. FRET-pairs suitable for use in the method of this invention.**

| **Donor** | **Acceptor** |
|---|---|
| **(a) Fluorescent donors** | |
| Fluorescein | Tetramethylrhodamine |
| Fluorescein | Cy-3 |
| Fluorescein | Rox |
| EDANS | DABCYL |
| Dansyl | Fluorescein |
| Cy3 | Cy-5 |
| Tryptophan | AEDANS |
| Fluorescein | Tetramethyl rhodamine |
| Tetramethyl rhodamine | DABCYL |
| Fluorescein | DABCYL |
| DABCYL | Cy-3 |
| Fluorescein | Hexachlorofluorescein |
| Tetrachlorofluorescein | Cy-5 |

| **(b) Luminescent donors** | |
|---|---|
| Europium | Cy-5 |
| Terbium | Tetramethyl rhodamine |
| Terbium | Cy-3 |

In a preferred embodiment, the donor-acceptor pair is a Fluorescein-ROX pair, e.g., FAM-ROX.

Reference herein to "fluorescence", "fluorescent dye" or "fluorescent groups" or "fluorophores" include luminescence, luminescent groups and suitable chromophores, respectively. In the present invention, the universal probe may be labeled with luminescent labels and luminescence resonance energy transfer is indicative of complex formation. Suitable luminescent probes include, but are not limited to, the luminescent ions of europium and terbium introduced as lanthium chelates (Heyduk & Heyduk, 1997). The lanthanide ions are also good donors for energy transfer to fluorescent groups (Selvin, 1995). Luminescent groups containing lanthanide ions can be incorporated into polynucleotides utilizing an 'open cage' chelator phosphoramidite. Table 6 gives some preferred luminescent groups.

In certain embodiments of the invention, the universal probes may also be labeled with two chromophores, and a change in the absorption spectra of the label pair is used as a detection signal, as an alternative to measuring a change in fluorescence.

There is a great deal of practical guidance available in the literature for selecting appropriate donor (receptor) -quencher pairs for particular probes, as exemplified by the following references: Clegg (1993, Proc. Natl. Acad. Sci., 90:2994-2998); Wu et al. (1994, Anal. Biochem., 218:1-13); Pesce et al., editors, Fluorescence Spectroscopy (1971, Marcel Dekker, New York); White et al., Fluorescence Analysis: A Practical Approach (1970, Marcel Dekker, New York); and the like. The literature also includes references providing exhaustive lists of fluorescent and chromogenic molecules and their relevant optical properties for choosing reporter-quencher pairs, e.g., Berlman, Handbook of Fluorescence Spectra of Aromatic Molecules, 2nd Edition (1971, Academic Press, New York); Griffiths, Colour and Constitution of Organic Molecules (1976, Academic Press, New York); Bishop, editor, Indicators (1972, Pergamon Press, Oxford); Haugland, Handbook of Fluorescent Probes and Research Chemicals (1992 Molecular Probes, Eugene) Pringsheim, Fluorescence and Phosphorescence (1949, Interscience Publishers, New York). Further, there is extensive guidance in the literature for derivatizing reporter and quencher molecules for covalent attachment via common reactive groups that can be added to an oligonucleotide, as exemplified by the following references, see, for example, Haugland (cited above); Ullman et al., U.S. Pat. No. 3,996,345; Khanna et al., U.S. Pat. No. 4,351,760; U.S. Patent Nos. 6,030,78, and 5,795,729.

Exemplary reporter-quencher pairs may be selected from xanthene dyes, including fluoresceins, and rhodamine dyes. Many suitable forms of these compounds are widely available commercially with substituents on their phenyl moieties which can be used as the site for bonding or as the bonding functionality for attachment to an oligonucleotide. Another group of fluorescent compounds are the naphthylamines, having an amino group in the alpha or beta position. Included among such naphthylamino compounds are 1-dimethylaminonaphthyl-5-sulfonate, 1-anilino-8-naphthalene sulfonate and 2-p-touidinyl-6-naphthalene sulfonate. Other dyes include 3-phenyl-7-isocyanatocoumarin, acridines, such as 9-isothiocyanatoacridine and acridine orange; N-(p-(2-benzoxazolyl)phenyl)maleimide; benzoxadiazoles, stilbenes, pyrenes, and the like.

In one embodiment, one universal probe is labeled with a dark quencher (e.g., a black hole quencher, BHQ) that absorbs or quenches fluorescence emitted by a receptor molecule (e.g., FAM). The BHQ dyes are a new class of dark quenchers that prevent fluorescence until a hybridization event occurs. In addition, these new dyes have no native fluorescence, virtually eliminating background problems seen with other quenchers. BHQ Dyes can be used to quench almost all reporter dyes and are commercially available, for example, from Biosearch Technologies, Inc (Novato, CA). The receptor fluorophore is used to label a chain terminator. The quencher molecule quenches the fluorescent signal emitted from the receptor molecule on the other universal probe when they are brought to close proximity, this results in a decrease in fluorescent signal generated by FRET.

Preferably, reporter molecules are fluorescent organic dyes derivatized for attachment to the terminal 3' carbon or terminal 5' carbon of the probe via a linking moiety. In some embodiments, quencher molecules are organic dyes, which may or may not be fluorescent, depending on the embodiment of the invention. Generally whether the quencher molecule is fluorescent or simply releases the transferred energy from the reporter by non-radiative decay, the absorption band of the quencher should substantially overlap the fluorescent emission band of the reporter molecule. Non-fluorescent quencher molecules that absorb energy from excited reporter molecules, but which do not release the energy radiatively, are referred to in the application as chromogenic molecules.

Fluorescein and rhodamine dyes and appropriate linking methodologies for attachment to oligonucleotides are described in many references, e.g., Marshall, Histochemical J., 7: 299-303 (1975); Menchen et al., U.S. Pat. No. 5,188,934; Menchen et al., European Patent Application 87310256.0; and Bergot et al., International Application PCT/US90/05565.

There are many linking moieties and methodologies for attaching labeling molecules (e.g., a member of an interactive labels) to the 5' or 3' termini of oligonucleotides, as exemplified by the following references: Eckstein, editor, Oligonucleotides and Analogues: A Practical Approach (IRL Press, Oxford, 1991); Zuckerman etal., Polynucleotides Research, 15: 5305-5321 (1987) (3' thiol group on oligonucleotide); Sharma et al., Polynucleotides Research, 19: 3019 (1991) (3' sulfhydryl); Giusti et al., PCR Methods and Applications. 2: 223-227 (1993) and Fung et al., U.S. Pat. No. 4,757,141 (5' phosphoamino group via Aminolink.TM. II available from Applied Biosystems, Foster City, Cafil.) Stabinsky, U.S. Pat. No. 4.739,044 (3' aminoalkylphosphoryl group); Agrawal et al., Tetrahedron Letters, 31: 1543-1546 (1990) (attachment via phosphoramidate linkages); Sproat et al., Polynucleotides Research, 15: 4837 (1987) (5' mercapto group); Nelson et al., Polynucleotides Research, 17: 7187-7194 (1989) (3' amino group); and the like.

A universal probe may be linked to a member of a pair of interactive labels at its 5' or 3' end. The 3' terminal of the oligonucleotide probe is blocked by a phosphate to prevent probe-initiated template independent elongation.

### Measurable Changes

In the method of the present invention, the labeled universal probes are capable of hybridizing to the target-hybridizing probes which bind to the target polynucleotide. These interactions lead to the formation of a complex in which the two labeled universal probes are brought to close proximity. The labels on the two universal probes, e.g., the donor-acceptor pair (e.g., a FRET fluorescent dye pair) are therefore brought into close proximity. Excitation of the donor causes emission on light with a higher wavelength, which in turn will excite the acceptor. The acceptor emits light with a higher wavelength than the exciting length's wavelength. This results in altered fluorescence of the complex compared to the uncomplexed fluorescence exhibited by the universal probes themselves when free in solution.

In one embodiment of the invention, fluorescence intensity of the universal probe and the fluorescence intensity of the complex are measured at one or more wavelengths with a fluorescence spectrophotometer, microtitre plate reader or real time PCR instruments. It is generally preferred that the two universal probes form a one - to - one complex and equal molar concentrations of the universal probes are present in the binding reaction. However, an excess of one probe may be used without departing from the scope of the invention.

Typically, it is preferable to look for a signal (a positive), rather than for the absence of a signal (a negative) in an assay of the invention, but it will be appreciated that either or both may be followed. The preferred method for generating a detectable signal, according to the invention, is FRET. The advantage to FRET is that a new light wavelength is created. It is easier to detect a small signal above background than to detect a small decrease in a large signal. If future energy transfer reactions were to be developed, such as magnetic resonance energy transfer, or biological resonance energy transfer (as between green fluorescent protein and luciferase), such processes could also be used.

In some embodiments of the invention, signal generated by FRET is detected by steady state measurements of the integrated emission intensity of the donor (i.e., the fluorescent dye that is excited by the light source used in the spectral measurement) and/or the acceptor (i.e., the fluorescent dye which has a absorption spectrum that overlaps the emission spectrum of the donor). In addition, FRET may be detected by time - resolved measurements in which the decay of donor fluorescence is measured after a short pulse of excitation. In certain embodiments of the invention the donor is excited at a wavelength that does not itself result in efficient excitation of the acceptor, and FRET is detected by measuring the excitation of the acceptor due to transfer of a photon from the donor.

In some embodiments, the signal is generated by quenching and then detected by fluorescent readers. Any FRET (e.g., black hole) or non-FRET (e.g., Dabcyl) quenchers may be used as quencher-reporter pair for the present invention.

In some embodiments of the invention, the donor-acceptor pair is replaced by a receptor-quencher pair. It is not critical to the invention which of the universal probes is labeled with a quenching molecule so long as the other is labeled with a corresponding receptor molecule of a receptor-quencher pair. Probes can be developed where the intensity of the reporter molecule fluorescence increases due to the separation of the reporter molecule from the quencher molecule. Probes can also be developed which lose their fluorescence because the quencher molecule is brought into proximity with the reporter molecule. These reporter--quencher molecule pair probes can be used in the universal probe system of the present invention to detect a target polynucleotide by monitoring either the appearance or disappearance of the fluorescence signal generated by the reporter molecule.

In one embodiment of the invention, the change of signal is measured using a spectrofluorophotometer.

### Chain Elongation-Primer Extension

In one embodiment of the invention, a target polynucleotide is subject to an amplification reaction before it amount or the amount of its amplified product is being detected. The amplification reaction employed in the subject methods is preferably catalyzed by a DNA polymerase. The reaction may be carried out by methods well known in the art, for example, as described in Current Protocols in Molecular Biology (1997, Ausubel et al., John Weley & Sons, Inc.).

The target-binding probes and the universal probes for the detection of a target polynucleotide may be added before or after the amplification reaction starts. Or it may be added at the end of each cycle of the amplification reaction if it is one similar to polymerase chain reaction (PCR).

Preferably, the target-binding probes and the universal probes are added before the amplification reaction starts.

In one embodiment, the amplification reaction is a PCR reaction and the PCR program may be set up so that the hybridization between the target-hybridizing probes are preloaded with their corresponding universal probes before the actual target amplification occurs. This may be achieved by any suitable methods known in the art, for example, by delaying the primer addition to the reaction mixture till after the annealing of the target-hybridizing probes and the universal probes occur, or by setting up an annealing temperature which allows the annealing of the probes but not the primers or the target-binding probes to the target. Therefore, it is preferred in this case to design the sequences of the universal probes so that they have a higher melting temperature (e.g., 1°C, or 2°C, or 5°C, or 10°C or higher) than the target-hybridizing probes and the primers.

To ensure the hybridization between the target-hybridizing probes and the universal probes, one can also use sequences having differential thermal stability. For example, the probe-binding sequence of the target-hybridizing probe can be chosen to have greater G/C content and, consequently, greater thermal stability than the target-binding sequence of the target-hybridizing probe. In similar fashion, one can incorporate modified nucleotides into the probe-binding sequence, which modified nucleotides contain base analogs that form more stable base pairs than the bases that are typically present in naturally occurring nucleic acids.

Modifications of the probe-binding sequence that may facilitate probe-probe binding prior to probe-primer binding to maximize the efficiency of the present assay include the incorporation of positively charged or neutral phosphodiester linkages in the probe to decrease the repulsion of the polyanionic backbones of the probe and target (see Letsinger et al., 1988, J. Amer. Chem. Soc. 110:4470); the incorporation of alkylated or halogenated bases, such as 5-bromouridine, in the probe-binding sequence to increase base stacking; the incorporation of ribonucleotides into the probe-binding sequence to force the probe:probe duplex into an "A" structure, which has increased base stacking; and the substitution of 2,6-diaminopurine (amino adenosine) for some or all of the adenosines in the probe-binding sequence.

In addition, to favor binding of the universal probes, before the primers, to the target-hybridizing probes, a high molar excess of universal probes may be used compared to the primers. One skilled in the art may also recognize that oligonucleotide concentration, length, and base composition are important factors that affect the Tm of any particular oligonucleotide in a reaction mixture. Each of these factors can be manipulated to create a thermodynamic bias to favor the universal probe annealing over primer annealing to the target-hybridizing probe.

In one embodiment, the primers, e.g., the forward and the reverse primers, are not added at the same concentration in the reaction mixture of the present invention. For example, if the target-hybridizing probe hybridizes to the target polynucleotide strand amplified by the forward primer, the forward primer is added at a higher concentration than the reverse primer which amplifies the other target polynucleotide strand which is not being hybridized by the target-hybridizing probe. On the other hand, if the target-hybridizing probe hybridizes to the target polynucleotide strand amplified by the reverse primer, the reverse primer is added at a higher concentration than the forward primer which amplifies the other target polynucleotide strand which is not being hybridized by the target-hybridizing probe.

In one embodiment, the primer which amplifies the probe-hybridizing target strand is added with a 5:1 ratio to the other primer which amplified the other target strand that does not hybridize to the probe.

After or during the amplification reaction, the target-hybridizing probes bind to the target polynucleotide and/or its amplified products. The preloaded universal probes, or the universal probes which bind to the target-hybridizing probes during or after the amplification, are then brought together by the two target-hybridizing probes which bind the target polynucleotide in close proximity. The labels on the universal probes, for example, a donor-acceptor pair, are therefore also in close proximity. The donor is excited by an applied wavelength, the donor causes emission on light with a higher wavelength, which in turn will excite the acceptor. The acceptor emits light with a higher wavelength than the exciting length's wavelength. This results in altered fluorescence which can is detectable by FRET assays.

### Hybridization

In the embodiments of the present invention, the target-hybridizing probes hybridize to the target polynucleotide or its amplified products, the universal probes hybridize to the target-hybridizing probes. In some embodiments where the target polynucleotide is amplified first, the primers used for the amplification also need to hybridize to the target polynucleotide template in order to initiate the amplification reaction.

Polynucleotide hybridization involves providing denatured polynucleotides (e.g., the target-hybridizing probe and the universal probe) under conditions where the two complementary (or partially complementary) polynucleotides can form stable hybrid duplexes through complementary base pairing. The polynucleotides that do not form hybrid duplexes optionally may be then washed away leaving the hybridized polynucleotides to be detected, typically through detection of an attached detectable label. Alternatively, the hybridization may be performed in a homogenous reaction in which all reagents are present at the same time and no washing is involved. In a preferred embodiment, two universal probes hybridize to two target-binding probes, each target-hybridizing probe comprise a target-binding sequence which binds the target polynucleotide and a probe binding sequence which binds to a universal probe, where the two target-hybridizing probes hybridize to the same strand of the target polynucleotide in close proximity. Preferably, the target-hybridizing probe which binds closer to the 3' end of the target strand comprises the target-binding sequence at 5' of the probe binding sequence, and the target-hybridizing probe which binds closer to the 5' end of the target strand comprises the target-binding sequence at 3' of the probe binding sequence so that the hybridization of the two target-hybridizing probes to the target polynucleotide results in the formation of a hybrid complex as shown in Figure 1. Also preferable, the two universal probes which hybridize tot eh two target-hybridizing probes are labeled at distinct end, e.g., one is labeled at 3' end and the other labeled at 5' end. This way, the hybridizing of the two universal probes to the target-hybridizing probe/target polynucleotide hybrid complex results in the two labels on the two universal probes being brought into close proximity for the purpose of generating a detectable signal. The unhybridized probes may be washed away, although this is not required for carrying out the present invention.

It is generally recognized that polynucleotides are denatured by increasing the temperature or decreasing the salt concentration of the buffer containing the polynucleotides. The stringency required is nucleotide sequence dependent and also depends upon the various components present during hybridization and/or washing. In some embodiments where the preload of the universal probes to the target-hybridizing probes are desirable, high stringent hybridization/washing conditions are used. Preferably, the probes are preloaded in a homogenous aqueous solution containing the target polynucleotide, where the preloading is achieved by designing the non-target-hybridizing probes to have higher Tms than the target-binding domain of the target-hybridizing probes.

Under high stringency conditions, majority of the hybridization occurs only between molecules which comprise complementary sequences, such as between a target-hybridizing probe which comprises a target-binding sequence and a probe binding sequence and a universal probe which hybridizes to the probe binding sequence of the target-hybridizing probe. However, it is not required two molecules to be completely complementary in order to hybridize under high stringency conditions. Under low stringency conditions (e.g., low temperature and/or high salt) hybrid duplexes will form even where the annealed sequences are not perfectly complementary. Thus specificity of hybridization is reduced at lower stringency. Conversely, at higher stringency (e.g., higher temperature or lower salt) successful hybridization requires fewer mismatches. In one embodiment, the hybridization between the target-hybridizing probes and the target polynucleotide is conducted under low stringency conditions.

In one embodiment, the hybridization of the target-hybridizing probes and the universal probes is carried out before a target polynucleotide is added, i.e., the target-hybridizing probes are pre-loaded with the universal probes, e.g., before the amplification reaction starts.

In another embodiment, the universal probes are added after the target-hybridizing probe already hybridized to the target polypeptide, e.g., after the amplification reaction finishes or after a cycle of the amplification reaction finishes.

In yet another embodiment of the invention, the target-hybridizing probes and the universal probes are simply added into the amplification reaction mixture and the hybridization between the probes is performed during the amplification reaction (e.g., a PCR reaction). This provides a homogenous assay method which does not require the purification of the probe complex from unhybridized probes.

### Pre-treatment Before Measuring

In some embodiments, undesired labels that might cause high background or other problems during the measuring or analysis (e.g., unhybridized universal probes) may be optionally removed by several ways. The operability of the subject methods of the present invention is not dependent upon the precise method of removal. The separation of hybridized probe/target complex and unhybridized universal probes may be achieved in a variety of ways, including, but not limited to, electrophoresis, separation by binding to a solid phase via a binding moiety on one polynucleotide of the complex, e.g., a target-hybridizing probe, chromatography, and the like. Suitable electrophoretic detection and separation systems include systems designed for the simultaneous electrophoretic separation and detection of fluorescently labeled polynucleotides; e.g., automated DNA sequencers such as the PE Applied Biosystems (Foster City, Calif., USA) 310, 377, or 3700.

Any of a broad range of solid supports known in the art could effectively be used in methods of the invention. For example, streptavidin - coated solid supports are available commercially such as for example, streptavidin - coated magnetic beads available from Promega (Madison, Wis.) and streptavidin coated microtitre plates (Covalink) available from NUNC (Raskilde, Denmark) or Labsystems (Marlboro, Mass.).

Separation methodologies dependent on nonspecific physical - chemical properties may be employed. Preferred methodologies include those methodologies in which specific affinity interactions are utilized such as solid support based affinity chromatography.

The disclosure also relates to compositions for performing the subject methods as described herein above. The compositions of the invention include mixtures that are formed in the course of performing the methods of the invention or compositions that may be formed in the process of preparing to perform methods of the invention. Examples of the subject composition include mixtures comprising the combinations of a target-hybridizing probe and a universal probe which hybridize to the target-hybridizing probe. The universal probe may be labeled with a label which is capable of generating detectable signals. The target-hybridizing probe may comprise a target binding sequence and a probe binding sequence. The target binding sequence does not hybridize to the target polynucleotide and may be common for a number of target-hybridizing probes which contains different sequences of the target binding sequence and used for the detection of different target polynucleotides. The present invention, therefore, relates to a universal probe system for the detection of different target polynucleotides so that it is no longer necessary to prepare a unique labeled probe for each target detection. For example, according to the universal probe system of the present invention, two labeled universal probes can be used for the detection of any target polynucleotide, so long as a pair of corresponding target-hybridizing probes (unlabeled) can be designed according to the present invention. This universal probe system therefore eliminates the need of individually label a specific probe for the detection of a specific target polynucleotide, which is laborious and expensive.

The present composition can be applied to systems that do not involve amplification. In this case, primers for amplification reaction are not needed. In fact, the present invention does not even require that polymerization or amplification occur. In the absence of an amplification reaction, the target-hybridizing probes simply hybridize to a denatured target polynucleotide. The universal probes may be preloaded to the target-hybridizing probes as described above herein, or they can hybridize to the target polynucleotide/target-hybridizing probe complex afterwards.

### EXAMPLES

### Example 1

In this example, the following probes were added to a sample containing a plasmid DNA containing the target, mouse muscle nicotinic acetylcholin receptor, γ subunit:
B7-P1-53
   5'-tgggcaagccattgagtggatctaatgacaggtagaagacgtgctctagttac-phosphate-3' (SEQ ID NO. 1)
UP-A: 5'gtaactagagcacgtcttctacctgtcat-FAM (SEQ ID NO. 2)
B7-P2-50
   5'-tctgacgttatactcggttacggaagttttgacccggaggctttcacaga-phosphate-3' (SEQ ID NO. 3)
UP-B: 5'-ROX-acttccgtaaccgagtataacgtcaga-phosphate-3' (SEQ ID NO. 4)

B7-P1-53 and B7-P2-50 were two target-hybridizing probes, UP-A and UP-B were the corresponding non-target hybridizing probes used for the detection. The interactive labels FAM and ROX on the two non-target-hybridizing probes UP-A and UP-B interact with each other when the probes hybridize to the two target-hybridizing probes B7-P1-53 and B7-P2-50, which hybridize to the target polynucleotide encoding the mouse muscle nicotinic acetylcholin receptor, γ subunit. This interaction generates a signal detectable by FRET.

### Example 2

In this example, a PCR reaction was set up as follows:

| Components | Volume (µl) | |
|---|---|---|
| | | Final Concentration |
| H₂O | 12 | |
| 10 X Brilliant core PCR buffer | 5 | 1X |
| MgCl₂ (50 mM) | 5.5 | 5.5 mM |
| dNTP (20mM)) | 2 | 200 uM each |
| F-primer (5 µM) | 1 | 100 nM |
| R-primer (5 µM) | 5 | 500 nM |
| UPA-FAM (5 µM) | 2 | 200 nM |
| UPB-ROX (5 µM) | 4 | 400 nM |
| P1-53 (5 µM) | 1 | 100 nM |
| P2-50 (5 uM) | 2 | 200 nM |
| SureStart Taq 5U/µl | 0.5 | 2.5 U |
| Total | 40 | |

The above reaction mixture was brought to a final volume of 50 µl by adding either 10 µl TE buffer or 10 µl TE buffer containing a plasmid DNA containing the target, mouse muscle nicotinic acetylcholin receptor, γ subunit. In this mixture, the ratios are: forward primer: reverse primer = 1 : 5; and P1:P2=1:2, UP-A:P1=2:1, UP-B:P2=2:1.

The PCR reaction was performed in a thermocycler according to the following profile: 95°C 10 min followed by 40 cycles of 95°C 30 sec, 55°C 1 min, 72°C 30 sec. The signals were collected at 55°C of each cycle.

The primers and probes used were the following:
Forward primer
   F-B7: cccagacttacagcaccag (SEQ ID NO. 5)
Reverse primer
   R-B7: gagtccaggagcattttagc (SEQ ID NO. 6)
B7-P1-53
   5'-tgggcaagccattgagtggatctaatgacaggtagaagacgtgctctagttac-phosphate-3' (SEQ ID NO. 7)
UP-A: 5'gtaactagagcacgtcttctacctgtcat-FAM (SEQ ID NO. 8)
B7-P2-50
   5'-tctgacgttatactcggttacggaagttttgacccggaggctttcacaga-phosphate-3' (SEQ ID NO. 9)
UP-B: 5'-ROX-acttccgtaaccgagtataacgtcaga-phosphate-3' (SEQ ID NO. 10)

As shown in Figure 2A, the fluorescent signals remained unchanged when no target template was added (NTC). The fluorescent signal increased as PCR cycle proceed when the plasmid DNA containing the target, mouse muscle nicotinic acetylcholin receptor, γ subunit was added. The increased signals were proportional to the target concentration. Figure 2B shows the linear responses between Ct and log concentration of target template using a universal probe system and the mouse muscle nicotinic acetylcholin receptor, g subunit target.

### OTHER EMBODIMENTS

The foregoing examples demonstrate experiments performed and contemplated by the present inventors in making and carrying out the invention. It is believed that these examples include a disclosure of techniques which serve to both apprise the art of the practice of the invention and to demonstrate its usefulness. It will be appreciated by those of skill in the art that the techniques and embodiments disclosed herein are preferred embodiments only that in general numerous equivalent methods and techniques may be employed to achieve the same result.

All of the references, including patents and patent applications, identified hereinabove, describe, set forth, provide a basis for or enable compositions and/or methods which may be important to the practice of one or more embodiments of the present inventions.

## Claims

1. A composition comprising:
- a polynucleotide target;
- a first target-hybridizing probe which comprises a target binding sequence (P1-DNA) which hybridizes to a strand of said target polynucleotide and a probe binding sequence (P1-P);
- a second target-hybridizing probe which comprises a target binding sequence (P2-DNA) which hybridizes, in close proximity to said first target-hybridizing probe and which hybridizes to the same strand of said target polynucleotide as said first target-hybridizing probe and wherein the second target-hybridizing probe further comprises a probe binding sequence (P2-P);
- a non-target-hybridizing probe 3 labeled with label A; and
- a non-target-hybridizing probe 4 labeled with label B, wherein said probe 3 hybridizes to said P1-P sequence and said probe 4 hybridizes to said P2-P sequence and wherein said label A and label B are interactive labels.

2. The composition according to claim 1, further **characterized in that**:
- said target binding sequence is at 5' of said probe binding in said probe 1, while said target binding sequence is at 3' of said probe binding sequence in said probe 2,
- said composition further comprises a forward and a reverse primer for the amplification of said target polynucleotide, and/or
- said composition further comprises a control polynucleotide.

3. The composition according to claim 2, wherein when said P1-DNA and P2-DNA bind to the strand amplified by said reverse primer, the amount of said forward primer to the amount of said reverse primer in said composition is 1 : 5; and when said P1-DNA and P2-DNA binds to the strand amplified by said forward primer, the amount of said forward primer to the amount of said reverse primer is 5 : 1.

4. The composition according to any of claims 1 to 3, further **characterized in that**:
- said label A and label B are fluorescent dyes,
- said label A and label B are a donor-acceptor pair which interact with each other to generate a signal by fluorescent resonance energy transfer (FRET),
- said probe 3 or 4 shares no homology to any polynucleotide isolated from a sample containing said target polynucleotide,
- said probe 3 is labeled at its 3' terminal and said probe 4 is labeled at its 5' terminal,
- the 3' terminal of a probe is modified to prevent probe extension,
- said 3' terminal of a probe is phosphorylated, and/or
- said probe 3 has a higher melting point (Tm) than said P1-DNA sequence, and said probe 4 has a higher Tm than said P2-DNA sequence.

5. The composition according to claim 4, further **characterized in that**:
- said donor-acceptor pair is a FAM/ROX pair, and/or
- said acceptor is a dark quencher.

6. A kit comprising:
- a polynucleotide target;
- a first target-hybridizing probe which comprises a target binding sequence (P1-DNA) which hybridizes to a strand of said target polynucleotide and a probe binding sequence (P1-P);
- a second target-hybridizing probe which comprises a target binding sequence (P2-DNA) which hybridizes, in close proximity to said first target-hybridizing probe and which hybridizes to the same strand of said target polynucleotide as said first target-hybridizing probe and wherein the second target-hybridizing probe further comprises a probe binding sequence (P2-P)
- a non-target-hybridizing probe 3 labeled with label A;
- a non-target-hybridizing probe 4 labeled with label B, wherein said probe 3 hybridizes to said P1-P sequence and said probe 4 hybridizes to said P2-P sequence and wherein said label A and label B are interactive labels; and
- packaging materials therefore.

7. The kit according to claim 6, further **characterized in that**:
- the 3' terminal of a probe is modified to prevent probe extension,
- said 3' terminal of a probe is phosphorylated,
- said kit further comprises a forward and a reverse primer for the amplification of said target polynucleotide,
- said kit further comprises a control polynucleotide, and/or
- said target binding sequence is at 5' of said probe binding in said probe 1, while said target binding sequence is at 3' of said probe binding in said probe 2.

8. The kit according to claim 7, wherein when said P1-DNA and P2-DNA binds to the strand amplified by said reverse primer, the amount of said forward primer to the amount of said reverse primer in said kit is 1: 5; and when said P1-DNA and P2-DNA binds to the strand amplified by said forward primer, the amount of said forward primer to the amount of said reverse primer is 5 : 1.

9. The kit according to any of claims 7 to 8, further **characterized in that**:
- said label A and label B are fluorescent dyes,
- said label A and label B are a donor-acceptor pair which interact with each other
- to generate a signal by fluorescent resonance energy transfer (FRET),
- said probe 3 or 4 shares no homology to any polynucleotide isolated from a sample containing said target polynucleotide,
- said probe 3 is labeled at its 3' terminal and said probe 4 is labeled at its 5' terminal, and/or said
- said probe 3 has a higher melting point (Tm) than said P1-DNA sequence, and said probe 4 has a higher Tm than said P2-DNA sequence.

10. The kit according to claim 9, further **characterized in that**:
- said donor-acceptor pair is a FAM/ROX pair, and/or
- said acceptor is a dark quencher.

11. A method for detecting the amount of a target polynucleotide, comprising:
(a) adding to said target polynucleotide:
(1) a target-hybridizing probe 1 comprising a target binding sequence (P1-DNA) which hybridizes to one strand of said target polynucleotide and a probe binding sequence (P1-P),
(2) a target-hybridizing probe 2 comprising a target binding sequence (P2-DNA) which hybridizes, in close proximity to said first target-hybridizing probe and which hybridizes to the same strand of said target polynucleotide as said first target-hybridizing probe and a probe binding sequence (P2-P) ;
(3) a non-target-hybridizing probe 3 labeled with label A which hybridizes to said P1-P sequence, and
(4) a non-target-hybridizing probe 4 labeled with label B which hybridizes to said P2-P sequence, wherein said addition permits said label A to interact with said label B to generate a detectable signal; and
(b) detecting said generated signal as indicative of the amount of said polynucleotide.

12. A method for detecting the amount of a target polynucleotide in an amplification reaction mixture, comprising:
(a) adding to said amplification reaction mixture:
(1) a target-hybridizing probe 1 comprising a target binding sequence (P1-DNA) which hybridizes to one strand of said target polynucleotide and a probe binding sequence (P1-P),
(2) a target-hybridizing probe 2 comprising a target binding sequence (P2-DNA) which hybridizes, in close proximity to said first target-hybridizing probe and which hybridizes to the same strand of said target polynucleotide as said first target-hybridizing probe and a probe binding sequence (P2-P),
(3) a non-target-hybridizing probe 3 labeled with label A which hybridizes to said P1-P sequence, and
(4) a non-target-hybridizing probe 4 labeled with label B which hybridizes to said P2-P sequence, wherein said addition permits said label A to interact with said label B to generate a signal; and
(b) detecting said generated signal as indicative of the amount of said polynucleotide.

13. The method according to claim 12, further **characterized in that**:
- when said P1-DNA and P2-DNA bind to the strand amplified by a reverse primer, the amount of a forward primer to the amount of said reverse primer in said reaction mixture is 1 : 5; and when said P1-DNA and P2-DNA bind to the strand amplified by a forward primer, the amount of said forward primer to the amount of a reverse primer is 5 : 1, and/or
- said amplification reaction is a polymerase chain reaction (PCR).

14. The method according to claim 13, wherein said generated detectable signal is detected at the end of two or more PCR cycles.

15. The method according to any of claims 11 to 14, further **characterized in that**:
- said label A and label B are fluorescent dyes,
- said label A and label B are a donor-acceptor pair which interact with each other to generate a signal by fluorescent resonance energy transfer (FRET),
- said probe 3 or 4 shares no homology to any polynucleotide isolated from a sample containing said target polynucleotide,
- said probe 3 is labeled at its 3' terminal and said probe 4 is labeled at its 5' terminal,
- the 3' terminal of a probe is modified to prevent probe extension,
- said 3' terminal of a probe is phosphorylated,
- said target binding sequence is at 5' of said probe binding in said probe1, while said target binding sequence is at 3' of said probe binding in said probe 2,
- said probe 3 has a higher melting point (Tm) than said P1-DNA sequence, and said probe 4 has a higher Tm than said P2-DNA sequence, and/or
- said generated signal is detected and compared with signals generated by one or more reference containing a known amount of said target polynucleotide for the determination of the amount of said target polynucleotide.

16. The method according to claim 15, further **characterized in that**:
- said donor-acceptor pair is a FAM/ROX pair, and/or
- said acceptor is a dark quencher.

## Patentansprüche

1. Zusammensetzung umfassend:
- ein Polynucleotid-Target;
- eine erste Target hybridisierende Sonde, die eine Target-Bindungssequenz (P1-DNA), die an einen Strang des Target-Polynucleotids hybridisiert, und eine Sondenbindungssequenz (P1-P) umfasst;
- eine zweite Target hybridisierende Sonde, die eine Target-Bindungssequenz (P2-DNA) umfasst, die in nächster Nähe zu der ersten Target hybridisierenden Sonde hybridisiert und die an denselben Strang des Target-Polynucleotids wie die erste Target hybridisierende Sonde hybridisiert und wobei die zweite Target hybridisierende Sonde des Weiteren eine Sondenbindungssequenz (P2-DNA) umfasst;
- eine Nicht-Target hybridisierende Sonde 3, die mit dem Label A gelabelt ist; und
- eine Nicht-Target hybridisierende Sonde 4, die mit dem Label B gelabelt ist, wobei die Sonde 3 an die P1-P-Sequenz hybridisiert und die Sonde 4 an die P2-P-Sequenz hybridisiert und wobei das Label A und das Label B interaktive Label sind.

2. Zusammensetzung nach Anspruch 1, des Weitere **gekennzeichnet dadurch, dass**:
- die Target-Bindungssequenz sich bei 5' der Sondenbindung in der Sonde 1 befindet, während die Target-Bindungssequenz sich bei 3' der Sondenbindungssequenz in der Sonde 2 befindet,
- die Zusammensetzung des Weiteren einen Vorwärts- und einen Reverseprimer für die Amplifizierung des Targetpolynucleotids umfasst und/oder
- die Zusammensetzung des Weiteren ein Kontrollpolynucleotid umfasst.

3. Zusammensetzung nach Anspruch 2, wobei, wenn die P1-DNA und die P2-DNA sich an den Strang binden, der durch den Reverseprimer amplifiziert ist, die Mengen des Vorwärtsprimers zur Menge des Reverseprimers in der Zusammensetzung 1: 5 beträgt, und wenn die P1-DNA und die P2-DNA sich an den Strang, binden, der durch den Vorwärtsprimer amplifiziert ist, die Menge des Vorwärtsprimers zur Menge des Reverseprimers 5:1 beträgt.

4. Zusammensetzung nach einem der Ansprüche 1 bis 3, des Weiteren **gekennzeichnet dadurch, dass**:
- das Label A und das Label B fluoreszierende Farbstoffe sind,
- das Label A und das Label B ein Donator-Akzeptorpaar sind, die mit einander in Wechselwirkung stehen, um durch Fluoreszenzresonanzenergieübertragung (FRET) ein Signal zu erzeugen,
- die Sonde 3 oder 4 keine Homologie mit irgendeinem Polynucleotid gemeinsam hat, das von einer Probe isoliert worden ist, die das Target-Polynucleotid enthält,
- wobei die Sonde 3 an ihrem 3'-Ende gelabelt ist und die Sonde 4 an ihrem 5'-Ende gelabelt ist,
- das 3'-Ende einer Sonde modifiziert ist, um die Sondenverlängerung zu verhindern,
- das 3'-Ende einer Sonde phosphoryliert ist und/oder
- die Sonde 3 einen höheren Schmelzpunkt (Tm) als die P1-DNA-Sequenz aufweist und die Sonde 4 eine höhere Tm aufweist als die P2-DNA-Sequenz.

5. Zusammensetzung nach Anspruch 4, des Weiteren **gekennzeichnet dadurch, dass**:
- das Donator-Akzeptorpaar ein FAM/ROX-Paar ist und/oder
- der Akzeptor ein dunkler Löscher ist.

6. Kit umfassend:
- ein Polynucleotid-Target;
- eine erste Target hybridisierende Sonde, die eine Target-Bindungssequenz (P1-DNA), die an einen Strang des Target-Polynucleotids hybridisiert, und eine Sondenbindungssequenz (P1-P) umfasst;
- eine zweite Target hybridisierende Sonde, die eine Target-Bindungssequenz (P2-DNA) umfasst, die in nächster Nähe zur ersten Target hybridisierenden Sonde hybridisiert und die an denselben Strang des Target-Polynucleotids wie die erste Target hybridisierende Sonde hybridisert und wobei die zweite Target hybridisierende Sonde des Weiteren eine Sondenbindungssequenz (P2-P) umfasst;
- eine Nicht-Target hybridisierende Sonde 3, die mit dem Label A gelabelt ist;
- eine Nicht-Target hybridisierende Sonde 4, die mit dem Label B gelabelt ist, wobei die Sonde 3 an die P1-P-Sequenz hybridisiert und die Sonde 4 an die P2-P-Sequenz hybridisiert und wobei das Label A und das Label B interaktive Label sind; und
- Verpackungsmaterialien dafür.

7. Kit nach Anspruch 6, des Weiteren **dadurch gekennzeichnet, dass**:
- das 3'-Ende einer Sonde modifiziert wird, um die Sondenverlängerung zu verhindern,
- das 3'-Ende einer Sonde phosphoryliert wird,
- wobei der Kit des Weiteren einen Vorwärts- und einen Reverseprimer für die Amplifizierung des Targetpolynucleotids umfasst,
- wobei der Kit des Weiteren ein Kontrollpolynucleotid umfasst und/oder
- die Target-Bindungssequenz sich bei 5' der Sondenbindung der Sonde 1 befindet, während die Target-Bindungssequenz sich bei 3' der Sondenbindungssequenz in der Sonde 2 befindet.

8. Kit nach Anspruch 7, wobei, wenn die P1-DNA und die P2-DNA sich an den Strang binden, der durch den Reverseprimer amplifiziert ist, die Menge des Vorwärtsprimers zur Menge des Reverseprimers in dem Kit 1: 5 beträgt und wenn die P1-DNA und die P2-DNA sich an den Strang, binden, der durch den Vorwärtsprimer amplifiziert ist, die Menge des Vorwärtsprimers zur Menge des Reverseprimers 5:1 beträgt.

9. Kit nach einem der Ansprüche 7 bis 8, des Weiteren **gekennzeichnet dadurch, dass**:
- das Label A und das Label B fluoreszierende Farbstoffe sind,
- das Label A und das Label B ein Donator-Akzeptorpaar sind, die mit einander in Wechselwirkung stehen, um durch Fluoreszenzresonanzenergieübertragung (FRET) ein Signal zu erzeugen,
- wobei die Sonde 3 oder 4 keine Homologie mit irgendeinem Polynucleotid gemeinsam hat, das von einer Probe isoliert worden ist, die das Target-Polynucleotid enthält,
- wobei die Sonde 3 an ihrem 3'-Ende gelabelt ist und die Sonde 4 an ihrem 5'-Ende gelabelt ist und/oder
- die Sonde 3 einen höheren Schmelzpunkt (Tm) als die P1-DNA-Sequenz aufweist und die Sonde 4 eine höhere Tm als die P2-DNA-Sequenz aufweist.

10. Kit nach Anspruch 9, des Weiteren **dadurch gekennzeichnet, dass**:
- das Donator-Akzeptorpaar ein FAM/ROX-Paar ist und/oder
- der Akzeptor ein dunkler Löscher ist.

11. Verfahren für das Erfassen der Menge eines Target-Polynucleotids umfassend:
(a) das Zugeben zu dem Target-Polynucleotid:
(1) einer Target hybridisierenden Sonde 1, die eine Target-bindungssequenz (P1-DNA), die an einen Strang des Target-Polynucleotids hybridisiert, und eine Sondenbindungssequenz (P1-P) umfasst;
(2) einer Target hybridisierenden Sonde 2, die eine Target-Bindungssequenz (P2-DNA), die in nächster Nähe zur ersten Target hybridisierenden Sonde hybridisiert und die an denselben Strang des Target-Polynucleotids wie die erste Target hybridisierende Sonde hybridisiert und eine Sondenbindungssequenz (P2-P) umfasst;
(3) einer Nicht-Target hybridisierenden Sonde 3, die mit dem Label A gelabelt ist, das an die P1-P-Sequenz hybridisiert; und
(4) einer Nicht-Target hybridisierenden Sonde 4, die mit dem Label B gelabelt ist, das an die P2-P-Sequenz hybridisiert, wobei die Zugabe es erlaubt, dass das Label A mit dem Label B in Wechselwirkung steht, um ein erfassbares Signal zu erzeugen; und
(b) das Erfassen des erzeugten Signals, wie es für die Menge des Polynucleotids indikativ ist.

12. Verfahren für das Erfassen der Menge eines Target-Polynucleotids in einer Amplifikationsreaktionsmischung, umfassend:
(a) das Zugeben zu der Amplifikationsreaktionsmischung:
(1) einer Target hybridisierenden Sonde 1, die eine Target-Bindungssequenz (P1-DNA), die an einen Strang des Target-Polynucleotids hybridisiert, und eine Sondenbindungssequenz (P1-P) umfasst;
(2) einer Target hybridisierenden Sonde 2, die eine Target-Bindungssequenz (P2-DNA), die in nächster Nähe zur ersten Target-Bindungssonde hybridisiert und die an denselben Strang des Target-Polynucleotids wie die erste Target hybridisierende Sonde hybridisiert, und eine Sondenbindungssequenz (P2-P) umfasst;
(3) einer Nicht-Target hybridisierenden Sonde 3, die mit dem Label A gelabelt ist, das an die P1-P-Sequenz hybridisiert; und
(4) einer Nicht-Target hybridisierenden Sonde 4, die mit dem Label B gelabelt ist, das an die P2-P-Sequenz hybridisiert, wobei das Zugeben erlaubt, dass das Label A mit dem Label B in Wechselwirkung steht, um ein Signal zu erzeugen; und
(b) Erfassen des erzeugten Signals als für die Menge des Polynucleotids indikativ.

13. Verfahren nach Anspruch 12, des Weiteren **dadurch gekennzeichnet, dass**:
- wenn die P1-DNA und die P2-DNA sich an den Strang binden, der durch einen Reverseprimer amplifiziert ist, die Menge des Vorwärtsprimers zur Menge des Reverseprimers in der Reaktionsmischung 1: 5 beträgt und wenn die P1-DNA und die P2-DNA sich an den Strang, binden, der durch einen Vorwärtsprimer amplifiziert ist, die Menge des Vorwärtsprimers zur Menge des Reverseprimers 5:1 beträgt und/oder
- die Amplifikationsreaktion eine Polymerasekettenreaktion (PCR) ist.

14. Verfahren nach Anspruch 13, wobei das erzeugte erfassbare Signal am Ende von zwei oder mehreren PCR-Zyklen erfasst wird.

15. Verfahren nach einem der Ansprüche 11 bis 14, des Weiteren **gekennzeichnet dadurch, dass**:
- die Label A und B fluoreszierende Farbstoffe sind,
- die Label A und B ein Donator-Akzeptorpaar sind, die mit einander in Wechselwirkung stehen, um durch Fluoreszenzresonanzenergieübertragung (FRET) ein Signal zu erzeugen,
- wobei die Sonde 3 oder 4 keine Homologie mit irgendeinem Polynucleotid gemeinsam hat, das von einer Probe isoliert worden ist, die das Target-Polynucleotid enthält,
- wobei die Sonde 3 an ihrem 3'-Ende gelabelt ist und die Sonde 4 an ihrem 5'-Ende gelabelt ist,
- das 3'-Ende einer Sonde modifiziert ist, um die Sondenverlängerung zu verhindern,
- das 3'-Ende einer Sonde phosphoryliert ist,
- die Target-Bindungssequenz sich an 5' der Sondenbindung in der Sonde 1 befindet, während die Target-Bindungssequenz sich bei 3' der Sondenbindung in der Sonde 2 befindet,
- die Sonde 3 einen höheren Schmelzpunkt (Tm) als die P1-DNA-Sequenz aufweist und die Sonde 4 eine höhere Tm aufweist als die P2-DNA-Sequenz und/oder
- das erzeugte Signal erfasst und mit Signalen verglichen wird, die durch eine oder mehrere Bezugssubstanzen erzeugt werden, die eine bekannte Menge des Target-Polynucleotids für die Bestimmung der Menge des Target-Polynucleotids enthalten.

16. Verfahren nach Anspruch 15, des Weiteren **dadurch gekennzeichnet, dass**:
- das Donator-Akzeptorpaar ein FAM/ROX-Paar ist und/oder
- der Akzeptor ein dunkler Löscher ist.

## Revendications

1. Composition comprenant:
- un polynucléotide cible;
- une première sonde d'hybridation à la cible qui comprend une séquence de liaison à la cible (P1-ADN) qui s'hybride à un brin dudit polynucléotide cible et une séquence de liaison à la sonde (P1-P) ;
- une seconde sonde d'hybridation à la cible qui comprend une séquence de liaison à la cible (P2-ADN) qui s'hybride, à proximité étroite de ladite première sonde d'hybridation à la cible et qui s'hybride au même brin dudit polynucléotide cible que ladite première sonde d'hybridation à la cible et dans laquelle la seconde sonde d'hybridation à la cible comprend en outre une séquence de liaison à la sonde (P2-P) ;
- une sonde ne s'hybridant pas à la cible 3 marquée avec un marqueur A ; et
- une sonde ne s'hybridant pas à la cible 4 marquée avec un marqueur B, dans laquelle ladite sonde 3 s'hybride à ladite séquence P1-P et ladite sonde 4 s'hybride à ladite séquence P2-P et dans laquelle lesdits marqueur A et marqueur B sont des marqueurs interactifs.

2. Composition selon la revendication 1, **caractérisée en outre en ce que** :
- ladite séquence de liaison à la cible est en 5' de ladite séquence de liaison à la sonde dans ladite sonde 1, alors que ladite séquence de liaison à la cible est en 3' de ladite séquence de liaison à la sonde dans ladite sonde 2,
- ladite composition comprend en outre une amorce avant et une amorce inverse pour l'amplification dudit polynucléotide cible, et/ou
- ladite composition comprend en outre un polynucléotide de contrôle.

3. Composition selon la revendication 2, dans laquelle lesdits P1-ADN et P2-ADN se lient au brin amplifié par ladite amorce inverse, la quantité de ladite amorce avant par rapport à la quantité de ladite amorce inverse dans ladite composition est de 1 : 5 ; et lorsque lesdits P1-ADN et P2-ADN se lient au brin amplifié par ladite amorce avant, la quantité de ladite amorce avant par rapport à la quantité de ladite amorce inverse est de 5 : 1.

4. Composition selon l'une quelconque des revendications 1 à 3, **caractérisée en outre en ce que** :
- lesdits marqueur A et marqueur B sont des colorants fluorescents,
- lesdits marqueur A et marqueur B sont une paire donneur-accepteur et interagissent l'un avec l'autre pour générer un signal par transfert d'énergie par résonance de fluorescence (FRET),
- ladite sonde 3 ou ladite sonde 4 ne partage pas d'homologie avec un polynucléotide quelconque isolé d'un échantillon contenant ledit polynucléotide cible,
- ladite sonde 3 est marquée à son extrémité 3' terminale et ladite sonde 4 est marquée à son extrémité 5' terminale,
- l'extrémité 3' terminale d'une sonde est modifiée pour empêcher l'extension de sonde,
- ladite extrémité 3' terminale d'une sonde est phosphorylée, et/ou
- ladite sonde 3 a une température de dénaturation (Tm) supérieure à celle de ladite séquence P1-ADN, et ladite sonde 4 a une Tm supérieure à celle de ladite séquence P2-ADN.

5. Composition selon la revendication **4, caractérisée en outre en ce que**:
- ladite paire donneur-accepteur est une paire FAM/ROX, et/ou
- ledit accepteur est un extincteur sombre.

6. Kit comprenant :
- un polynucléotide cible ;
- une première sonde d'hybridation à la cible qui comprend une séquence de liaison à la cible (P1-ADN) qui s'hybride à un brin dudit polynucléotide cible et une séquence de liaison à la sonde (P1-P) ;
- une seconde sonde d'hybridation à la cible qui comprend une séquence de liaison à la cible (P2-ADN) qui s'hybride, à proximité étroite de ladite première sonde d'hybridation à la cible et qui s'hybride au même brin dudit polynucléotide cible que ladite première sonde d'hybridation à la cible et dans lequel la seconde sonde d'hybridation à la cible comprend en outre une séquence de liaison à la sonde (P2-P) ;
- une sonde ne s'hybridant pas à la cible 3 marquée avec un marqueur A ;
- une sonde ne s'hybridant pas à la cible 4 marquée avec un marqueur B, dans laquelle ladite sonde 3 s'hybride à ladite séquence P1-P et ladite sonde 4 s'hybride à ladite séquence P2-P et dans laquelle lesdits marqueur A et marqueur B sont des marqueurs interactifs ; et
- des matériaux d'emballage pour ceci.

7. Kit selon la revendication 6, **caractérisée en outre en ce que** :
- l'extrémité 3' terminale d'une sonde est modifiée pour empêcher l'extension de sonde,
- ladite extrémité 3' terminale d'une sonde est phosphorylée,
- ledit kit comprend en outre une amorce avant et une amorce inverse pour l'amplification dudit polynucléotide cible,
- ledit kit comprend en outre un polynucléotide de contrôle, et/ou
- ladite séquence de liaison à la cible est en 5' de ladite séquence de liaison à la sonde dans ladite sonde 1, alors que ladite séquence de liaison à la cible est en 3' de ladite séquence de liaison à la sonde dans ladite sonde 2,

8. Kit selon la revendication 7, dans lequel lesdits P1-ADN et P2-ADN se lient au brin amplifié par ladite amorce inverse, la quantité de ladite amorce avant par rapport à la quantité de ladite amorce inverse dans ledit kit est de 1 : 5 ; et lorsque lesdits P1-ADN et P2-ADN se lient au brin amplifié par ladite amorce avant, la quantité de ladite amorce avant par rapport à la quantité de ladite amorce inverse est de 5 : 1.

9. Kit selon l'une quelconque des revendications 7 à 8, **caractérisé en outre en ce que** :
- lesdits marqueur A et marqueur B sont des colorants fluorescents,
- lesdits marqueur A et marqueur B sont une paire donneur-accepteur et interagissent l'un avec l'autre pour générer un signal par transfert d'énergie par résonance de fluorescence (FRET),
- ladite sonde 3 ou ladite sonde 4 ne partage pas d'homologie avec un polynucléotide quelconque isolé d'un échantillon contenant ledit polynucléotide cible,
- ladite sonde 3 est marquée à son extrémité 3' terminale et ladite sonde 4 est marquée à son extrémité 5' terminale, et/ou
- ladite sonde 3 a une température de dénaturation (Tm) supérieure à celle de ladite séquence P1-ADN, et ladite sonde 4 a une Tm supérieure à celle de ladite séquence P2-ADN.

10. Kit selon la revendication 9, **caractérisé en outre en ce que** :
- ladite paire donneur-accepteur est une paire FAM/ROX, et/ou
- ledit accepteur est un extincteur sombre.

11. Procédé de détection de la quantité d'un polynucléotide cible, qui comprend:
(a) l'ajout au dit polynucléotide cible :
(1) d'une sonde d'hybridation à la cible 1 comprenant une séquence de liaison à la cible (P1-ADN) qui s'hybride à un brin dudit polynucléotide cible et une séquence de liaison à la sonde (P1-P),
(2) d'une sonde d'hybridation à la cible 2 comprenant une séquence de liaison à la cible (P2-ADN) qui s'hybride, à proximité étroite de ladite première sonde d'hybridation à la cible et qui s'hybride au même brin dudit polynucléotide cible que ladite première sonde d'hybridation à la cible et une séquence de liaison à la sonde (P2-P) ;
(3) une sonde ne s'hybridant pas à la cible 3 marquée avec un marqueur A qui s'hybride à ladite séquence P1-P, et
(4) une sonde ne s'hybridant pas à la cible 4 marquée avec le marqueur B qui s'hybride à ladite séquence P2-P, dans lequel ledit ajout permet que ledit marqueur A interagisse avec ledit marqueur B pour générer un signal détectable ; et
(b) la détection dudit signal généré en tant qu'un indicateur de la quantité dudit polynucléotide.

12. Procédé de détection de la quantité d'un polynucléotide cible dans un mélange réactionnel d'amplification, qui comprend :
(a) l'ajout au dit mélange réactionnel d'amplification :
(1) d'une sonde d'hybridation à la cible 1 comprenant une séquence de liaison à la cible (P1-ADN) qui s'hybride à un brin dudit polynucléotide cible et une séquence de liaison à la sonde (P1-P),
(2) d'une sonde d'hybridation à la cible 2 comprenant une séquence de liaison à la cible (P2-ADN) qui s'hybride, à proximité étroite de ladite première sonde d'hybridation à la cible et qui s'hybride au même brin dudit polynucléotide cible que ladite première sonde d'hybridation à la cible et une séquence de liaison à la sonde (P2-P),
(3) une sonde ne s'hybridant pas à la cible 3 marquée avec un marqueur A qui s'hybride à ladite séquence P1-P, et
(4) une sonde ne s'hybridant pas à la cible 4 marquée avec un marqueur B qui s'hybride à ladite séquence P2-P, dans lequel ledit ajout permet que ledit marqueur A interagisse avec ledit marqueur B pour générer un signal ; et
(b) la détection dudit signal généré en tant qu'un indicateur de la quantité dudit polynucléotide.

13. Procédé selon la revendication 12, **caractérisé en outre en ce que**:
- lorsque lesdits P1-ADN et P2-ADN se lient au brin amplifié par une amorce inverse, la quantité d'une amorce avant par rapport à la quantité de ladite amorce inverse est de 1 : 5 ; et lorsque lesdits P1-ADN et P2-ADN se lient au brin amplifié par une amorce avant, la quantité de ladite amorce avant par rapport à la quantité d'une amorce inverse est de 5 : 1. et/ou
- ladite réaction d'amplification est une réaction d'amplification en chaîne par polymérase (PCR).

14. Procédé selon la revendication 13, dans lequel ledit signal détectable généré est détecté à la fin de deux cycles de PCR ou plus.

15. Procédé selon l'une quelconque des revendications 11 à 14, **caractérisé en outre en ce que** :
- lesdits marqueur A et marqueur B sont des colorants fluorescents,
- lesdits marqueur A et marqueur B sont une paire donneur-accepteur et interagissent l'un avec l'autre pour générer un signal par transfert d'énergie par résonance de fluorescence (FRET),
- ladite sonde 3 ou ladite sonde 4 ne partage pas d'homologie avec un polynucléotide quelconque isolé d'un échantillon contenant ledit polynucléotide cible,
- ladite sonde 3 est marquée à son extrémité 3' terminale et ladite sonde 4 est marquée à son extrémité 5' terminale,
- l'extrémité 3' terminale d'une sonde est modifiée pour empêcher l'extension de sonde,
- ladite extrémité 3' terminale d'une sonde est phosphorylée,
- ladite séquence de liaison à la cible est en 5' de ladite liaison à la sonde dans ladite sonde 1, alors que ladite séquence de liaison à la cible est en 3' de ladite séquence de liaison à la sonde dans ladite sonde 2,
- ladite sonde 3 a une température de dénaturation (Tm) supérieure à celle de ladite séquence P1-ADN, et ladite sonde 4 a une Tm supérieure à celle de ladite séquence P2-ADN, et/ou
- ledit signal généré est détecté et comparé à des signaux générés par une ou plusieurs références contenant une quantité connue dudit polynucléotide cible pour la détermination de la quantité dudit polynucléotide cible.

16. Procédé selon la revendication 15, **caractérisé en outre en ce que** :
- ladite paire donneur-accepteur est une paire FAM/ROX, et/ou
- ledit accepteur est un extincteur sombre.
